# EUROPEAN PATENT APPLICATION

(11) **EP 4 032 907 A1**
(43) Date of publication of application: **27.07.2022**
(21) Application number: 19945687.2
(22) Date of filing: 20.09.2019
(51) Int. Cl.: C07K 16/28, C07K 16/18, C12N 5/20, A61K 39/395

(54) **BCMA-TARGETED ANTIBODY AND CHIMERIC ANTIGEN RECEPTOR**

(71) Applicant: Shanghai Genbase Biotechnology Co., Ltd., China (Shanghai) Pilot Free Trade Zone Shanghai 201203 (CN)
(72) Inventor: DU, Liang, Shanghai 201203 (CN); MOU, Nan, Shanghai 201203 (CN); ZHANG, Hongyan, Shanghai 201203 (CN); JIN, Lina, Shanghai 201203 (CN); YU, Yue, Shanghai 201203 (CN); YUAN, Jijun, Shanghai 201203 (CN)
(74) Representative: Papula Oy
(86) International application number: PCT/CN2019/107004
(87) International publication number: WO 2021/051390

(57) **Abstract**

An antibody specifically binding to BCMA and an antigen binding fragment thereof, a chimeric antigen receptor (CAR) containing the antigen binding fragment, a nucleic acid molecule encoding the CAR, an immune effect cell expressing the CAR, a method for preparing the immune effect cell, use of the CAR and the immune effect cell for preventing and/or treating a B cell-related disease (for example, a B-cell malignancy and an autoimmune disease), and a method for preventing and/or treating the B cell-related disease

## Description

### Technical Field

The present invention relates to the field of disease treatment and immunology; in particular, the present invention relates to an antibody or an antigen-binding fragment thereof that specifically binds to BCMA, and a chimeric antigen receptor (CAR) comprising the same. The present invention also relates to a nucleic acid molecule encoding the CAR, an immune effector cell expressing the CAR, and a method for preparing the immune effector cell. The present invention also relates to a use of the CAR and immune effector cell for preventing and/or treating a B cell-related disease (e.g., B cell malignancy, autoimmune disease) and a method for preventing and/or treating the B cell-related disease.

### Background Art

B cells are a kind of lymphocytes, derived from pluripotent stem cells of bone marrow, which play an important role in producing antibodies, mediating humoral immune responses, presenting soluble antigens, producing a large number of cytokines, participating in immune regulation, inflammatory responses and hematopoiesis^{[1]}. Some major diseases involve B cells. Malignant transformation of B cells leads to cancers, including some lymphomas such as Hodgkin's lymphoma and multiple myeloma. Abnormal B cell physiology may contribute to the development of autoimmune diseases, including systemic lupus erythematosus. Both of the above categories of diseases involving B cells can be considered as a result of B cells overgrowing and/or inappropriately attacking parts of the body, and a possible control strategy is the use of antibodies or antigen-binding fragments thereof that target pathological B cells, or other pharmaceutical forms based on these antibodies or antigen-binding fragments thereof.

B-cell maturation antigen (BCMA), also known as TNFRSF17 or CD269, consists of 184 amino acids and belongs to a type I transmembrane protein lacking a signal peptide. BCMA is a member of the tumor necrosis factor receptor family (TNFR) and binds the TNF family ligands BAFF (B-cell activating factor) and APRIL (proliferation-inducing ligand)^{[2]}. Analysis of various B cell lines shows that BCMA is expressed on the surface of mature B cells and plasma cells^{[3]}, and the studies about gene level/protein level in normal human tissues show that BCMA is not expressed in other human normal tissues, except mature B cells and plasma cells, and is not expressed in CD34+ hematopoietic cells^{[4]}. BCMA knockout mice have normal lymphoid organs and immune systems^{[5]}, with normal development of B lymphocytes, but the number of plasma cells is significantly reduced, which proves that BCMA plays an important role in maintaining the survival of plasma cells, and the mechanism mainly comprises the binding of BCMA to BAFF protein, transduction of signals to activate the NF-κB pathway, and up-regulation of anti-apoptotic genes Bcl-2, Mcl-1 and Bclw, etc., to maintain cell growth^{[6]}. Studies have shown that BCMA is ubiquitously expressed in B-cell malignancies such as multiple myeloma (MM) and non-Hodgkin's lymphoma (NHL), and plays an important role in promoting the malignant proliferation of tumor cells^{[7]}. In sum, BCMA can be used as one of the targets of B-cell malignancies for the treatment of multiple myeloma and non-Hodgkin's lymphoma.

The effect of BCMA-targeted therapy is first demonstrated in the progression of multiple myeloma. Multiple myeloma is a malignant plasma cell disease characterized by the clonal proliferation of malignant plasma cells within the bone marrow, which secrete monoclonal immunoglobulin or its fragment (M protein), resulting in damage of bone, kidney and other related target organs or tissues, and which is manifested as bone pain, anemia, renal insufficiency, infection, etc.^{[8]}. Currently, multiple myeloma is the second most common malignant tumor in the hematological system, accounting for 13% of the malignant tumor of the hematological system, and its incidence increases with age and has a tendency to occur in youngers in recent years^{[9]}. Currently, researchers are actively involved in developing three major types of immunotherapies targeting BCMA, which are chimeric antigen receptor T cells (CAR-T cells), bispecific antibodies and antibody drug conjugates (ADCs), and the results of preclinical and clinical studies demonstrate the safety and efficacy of these classes of therapies targeting BCMA.

The most advanced of these BCMA-targeting drugs is bb2121 of Blue Bird. The extracellular domain used in the pharmaceutical form of bb2121 comprises an antigen-binding fragment of mouse antibody that binds to human BCMA (CN 201580073309.6), which is derived from the anti-BCMA antibody with clone number C11D5.3 (CN 201510142069.2) developed by Biogen Idec. C11D5.3 is widely used in BCMA-CAR T cell therapy. In addition to bb2121, bb21217, BCMA-CAR T of NCI, and BCMA-CAR T (CN 201610932365.7) of Hengrun Dasheng Biotechnology Co., Ltd. all achieve the binding to BCMA target through the antigen-binding fragment of this clone, and all of them use the mouse-derived form of this clone.

Humanized antibodies can greatly reduce the immune side effects caused by heterologous antibodies to the human body. A classic method for humanization of murine antibodies is CDR grafting. The CDR of antibody variable region is the regions where the antibody recognizes and binds to the antigen, which directly determines the specificity of the antibody. By grafting the CDR of murine monoclonal antibody into the variable region of human antibody to replace the CDR of the human antibody, the human antibody can obtain the antigen-binding specificity of the murine monoclonal antibody while its heterogeneity is reduced. However, although the antigen mainly contacts the CDR of antibody, the FR region also often participates in the action, affecting the spatial configuration of the CDR. Therefore, after the replacement with human FR region, the V region in which the murine CDR and the human FR are embedded may change the CDR configuration of the single antigen, and the ability to bind the antigen will decrease or even significantly decrease; in addition, this may bring obviously undesired activity. For example, in the patent application (CN 201580050638.9) of Blue Bird, it was found that a CAR T constructed with humanized C11D5.3 as antigen-binding portion could cause significant antigen-independent cytokine release, and it was observed that the introduction of this humanized CAR could induce continuous activation and exhaustion of T cells. The structural modification of the parts other than the BCMA-binding portion of the CAR did not solve the above problems, making anti-BCMA CAR based on this humanized anti-Cl 1D5.3 unsuitable for T cell therapy.

### Contents of the present invention

The inventors of the present invention have conducted in-depth research and modification on the murine antibody C11D5.3 that specifically recognizes BCMA, and developed a humanized antibody of the murine antibody, which maintains a comparable activity of binding to BCMA-expressing tumor cells as compared with the murine antibody, and shows a significantly reduced non-specific binding to normal tissue cells. On this basis, the inventors have made a lot of creative work to further construct and obtain a chimeric antigen receptor (CAR) that can specifically bind to BCMA. The CAR of the present invention is capable of directing immune effector cell specificity and reactivity to BCMA-expressing cells in an MHC-non-restricted manner so as to clear the same, without causing antigen-independent cytokine release, and without inducing sustained activation and exhaustion of T cells. Therefore, the humanized antibodies and CARs of the present invention have the potential for preventing and/or treating B cell-related diseases (e.g., B cell malignancies, autoimmune diseases, etc.), and thus have great clinical values.

### Antibody of the present invention

Accordingly, in a first aspect, the present invention provides an antibody or antigen-binding fragment thereof capable of specifically binding to BCMA, the antibody or antigen-binding fragment thereof comprising:
(a) a heavy chain variable region (VH) comprising an amino acid sequence selected from the group consisting of:
   (i) a sequence set forth in SEQ ID NO: 1 or 3;
   (ii) a sequence having a substitution, deletion or addition of one or several amino acids (e.g., substitution, deletion or addition of 1, 2, 3, 4 or 5 amino acids) compared to the sequence set forth in SEQ ID NO: 1 or 3; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 1 or 3;
   and/or,
(b) a light chain variable region (VL) comprising an amino acid sequence selected from the group consisting of:
   (iv) a sequence set forth in SEQ ID NO: 2 or 4;
   (v) a sequence having a substitution, deletion or addition of one or several amino acids (e.g., substitution, deletion or addition of 1, 2, 3, 4 or 5 amino acids) compared to the sequence set forth in SEQ ID NO: 2 or 4; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 2 or 4.

In certain embodiments, the substitution described in (ii) or (v) is a conservative substitution.

In certain embodiments, the antibody or antigen-binding fragment thereof comprises:
(a) a heavy chain variable region (VH) comprising an amino acid sequence selected from the group consisting of:
   (i) a sequence set forth in SEQ ID NO: 1;
   (ii) a sequence having a substitution, deletion or addition of one or several amino acids (e.g., substitution, deletion or addition of 1, 2, 3, 4 or 5 amino acids) compared to the sequence set forth in SEQ ID NO: 1; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 1;
   and/or,
(b) a light chain variable region (VL) comprising an amino acid sequence selected from the group consisting of:
   (iv) a sequence set forth in SEQ ID NO: 2;
   (v) a sequence having a substitution, deletion or addition of one or several amino acids (e.g., substitution, deletion or addition of 1, 2, 3, 4 or 5 amino acids) compared to the sequence set forth in SEQ ID NO: 2; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 2.

In certain embodiments, the substitution described in (ii) or (v) is a conservative substitution.

In certain embodiments, the antibody or antigen-binding fragment thereof comprises a heavy chain variable region (VH) and a light chain variable region (VL), the VH comprising a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 2 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto.

In certain embodiments, the antibody or antigen-binding fragment thereof comprises:
(a) a heavy chain variable region (VH) comprising an amino acid sequence selected from the group consisting of:
   (i) a sequence set forth in SEQ ID NO: 3;
   (ii) a sequence having a substitution, deletion or addition of one or several amino acids (e.g., substitution, deletion or addition of 1, 2, 3, 4 or 5 amino acids) compared to the sequence set forth in SEQ ID NO: 3; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 3;
   and/or,
(b) a light chain variable region (VL) comprising an amino acid sequence selected from the group consisting of:
   (iv) a sequence set forth in SEQ ID NO: 4;
   (v) a sequence having a substitution, deletion or addition of one or several amino acids (e.g., substitution, deletion or addition of 1, 2, 3, 4 or 5 amino acids) compared to the sequence set forth in SEQ ID NO: 4; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 4.

In certain embodiments, the substitution described in (ii) or (v) is a conservative substitution.

In certain embodiments, the antibody or antigen-binding fragment thereof comprises a heavy chain variable region (VH) and a light chain variable region (VL), the VH comprising a sequence set forth in SEQ ID NO: 3 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 4 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto.

In certain embodiments, the antibody or antigen-binding fragment thereof has one or more of the following biological functions:
(a) binding to an isolated human BCMA protein with an affinity not lower than that of the murine antibody C11D5.3; wherein the expression "isolated" means that the protein is not contained in the cell or on the cell surface; and the affinity can be determined by, for example, the term ka (association rate constant of antibody from antibody/antigen complex), kD (dissociation constant) and/or KD (kD/ka);
(b) binding to an isolated human BCMA protein with an EC50 of 0.05 µg/ml or less (e.g., 0.04 µg/ml, 0.03 µg/ml, 0.02 µg/ml or less); for example, the EC50 is determined by an ELISA technique;
(c) binding to a human BCMA-expressing cell with an affinity not lower than that of the murine antibody C11D5.3; the affinity can be determined by, for example, the term ka (association rate constant of antibody from antibody/antigen complex), kD (dissociation constant) and/or KD (kD/ka);
(d) binding to a human BCMA-expressing cell with an EC50 of 8 µg/ml or less (e.g., 5 µg/ml, 4 µg/ml, 3 µg/ml, 2 µg/ml, 1 µg/ml or less); for example, the EC50 is measured by a flow cytometry;
(e) binding to a normal tissue (e.g., non-tumor tissue) cell with an affinity lower than that of the murine antibody C11D5.3;
(f) a T cell expressing a CAR comprising the antibody or antigen-binding fragment thereof would not substantially cause an antigen-independent cytokine release.

In certain embodiments, the antibody or antigen-binding fragment thereof of the present invention may further comprise a constant region sequence derived from a mammalian (e.g., murine or human) immunoglobulin, or variant thereof, wherein the variant has a substitution, deletion or addition of one or more amino acids compared to the sequence from which it is derived. In certain embodiments, the variant has a conservative substitution of one or more amino acids compared to the sequence from which it is derived.

In certain embodiments, the antibody or antigen-binding fragment thereof of the present invention has a heavy chain comprising a heavy chain constant region (CH) of a human immunoglobulin, or a variant thereof, wherein the variant has a substitution, deletion or addition of one or more amino acids (e.g., substitution, deletion or addition of up to 20, up to 15, up to 10, or up to 5 amino acids; e.g., substitution, deletion or addition of 1, 2, 3, 4 or 5 amino acids) compared to the sequence from which it is derived; and/or,

The antibody or antigen-binding fragment thereof of the present invention has a light chain comprising a light chain constant region (CL) of a human immunoglobulin, or a variant thereof, wherein the variant has a substitution, deletion or addition of up to 20 amino acids (e.g., substitution, deletion or addition of up to 15, up to 10, or up to 5 amino acids; e.g., substitution, deletion or addition of 1, 2, 3, 4 or 5 amino acids) compared to the sequence from which it is derived.

In certain embodiments, the heavy chain constant region is selected from IgG, IgM, IgE, IgD or IgA.

In certain embodiments, the heavy chain constant region is an IgG heavy chain constant region, for example, an IgGl, IgG2, IgG3 or IgG4 heavy chain constant region. In certain embodiments, the heavy chain constant region is a murine IgGl, IgG2, IgG3 or IgG4 heavy chain constant region. In certain embodiments, the heavy chain constant region is a human IgGl, IgG2, IgG3 or IgG4 heavy chain constant region. In certain embodiments, the heavy chain constant region is preferably a human IgGl or IgG4 heavy chain constant region.

In certain embodiments, the light chain constant region is selected from κ or λ.

In certain embodiments, the light chain constant region is a κ light chain constant region. In certain embodiments, the light chain constant region is a murine κ light chain constant region. In certain embodiments, the light chain constant region is a human κ light chain constant region.

In certain embodiments, the antibody of the present invention is a humanized antibody. In certain embodiments, the antigen-binding fragment of the present invention is selected from the group consisting of Fab, Fab', (Fab')₂, Fv, disulfide-linked Fv, scFv, di-scFv, (scFv)₂.

In the present invention, the antibody or antigen-binding fragment thereof of the present invention may comprises a variant, wherein the variant differs from the antibody or antigen-binding fragment from which it is derived only in a conservative substitution of one or more amino acid residues (e.g., conservative substitutions of up to 20, up to 15, up to 10, or up to 5 amino acids), or has a sequence identity of at least 85%, at least 90%, at least 95%, at least 96%, at least 97%, at least 98%, at least 99%, or 100% compared with the antibody or antigen-binding fragment thereof from which it is derived, and substantially retains the above-mentioned biological function of the antibody or antigen-binding fragment thereof from which it is derived.

### Preparation of antibody

The antibody of the present invention can be prepared by various methods known in the art, such as by genetic engineering recombinant techniques. For example, DNA molecules encoding the heavy and light chains of the antibody of the present invention are obtained by chemical synthesis or PCR amplification. The resulting DNA molecules are inserted into an expression vector and then transfected into a host cell. Then, the transfected host cells are cultured under specific conditions to express the antibody of the present invention.

The antigen-binding fragments of the present invention can be obtained by hydrolysis of intact antibody molecules (see Morimoto et al., J. Biochem. Biophys. Methods 24:107-117 (1992); and Brennan et al., Science 229:81 (1985)). Alternatively, these antigen-binding fragments can also be produced directly by recombinant host cells (reviewed in Hudson, Curr. Opin. Immunol. 11:548-557 (1999); Little et al., Immunol. Today, 21:364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the culture medium of recombinant host cells. Other techniques for preparing these antigen-binding fragments are well known to those of ordinary skill in the art.

Accordingly, in a second aspect, the present invention provides an isolated nucleic acid molecule, which comprises a nucleotide sequence encoding the antibody or antigen-binding fragment thereof of the present invention, or a heavy chain variable region and/or light chain variable region thereof. In certain preferred embodiments, the isolated nucleic acid molecule encodes the antibody or antigen-binding fragment thereof of the present invention, or a heavy chain variable region and/or light chain variable region thereof.

In a third aspect, the present invention provides a vector (e.g., cloning vector or expression vector), which comprises the isolated nucleic acid molecule of the present invention. In certain preferred embodiments, the vector of the present invention is, for example, plasmid, cosmid, phage, and the like. In certain preferred embodiments, the vector is capable of expressing the antibody or antigen-binding fragment thereof of the present invention in a subject (e.g., a mammal, for example, a human).

In a fourth aspect, the present invention provides a host cell, which comprises the isolated nucleic acid molecule of the present invention or the vector of the present invention. The host cell includes, but is not limited to, prokaryotic cell such as *E. coli* cell, and eukaryotic cell such as yeast cell, insect cell, plant cell, and animal cell (e.g., mammalian cell, for example, mouse cell, human cell, etc.). In certain preferred embodiments, the host cell of the present invention is a mammalian cell, for example, CHO (e.g., CHO-K1, CHO-S, CHO DG44).

In another aspect, there is also provided a method for preparing the antibody or antigen-binding fragment thereof of the present invention, which comprises, culturing the host cell of the fourth aspect under conditions that permit expression of the antibody or antigen-binding fragment thereof, and recovering the antibody or antigen-binding fragment thereof from a culture of the cultured host cell.

### Conjugate

The antibody or antigen-binding fragment thereof of the present invention can be derivatized, for example, linked to another molecule (e.g., another polypeptide or protein). In general, the derivatization (e.g., labeling) of an antibody or antigen-binding fragment thereof does not adversely affect its binding to BCMA, particularly human BCMA. Accordingly, the antibody or antigen-binding fragment thereof of the present invention are also intended to include such derivatization forms. For example, the antibody or antigen-binding fragment thereof of the present invention can be functionally linked (by chemical coupling, genetic fusion, non-covalent attachment, or otherwise) to one or more other molecular moieties, such as another antibody (e.g., to form a bispecific antibody), detection reagent, pharmaceutical reagent, and/or protein or polypeptide (e.g., avidin or polyhistidine tag) capable of mediating the binding of the antibody or antigen-binding fragment thereof to another molecule.

Accordingly, in a fifth aspect, the present invention provides a conjugate, which comprises the antibody or antigen-binding fragment thereof of the present invention and a modification moiety linked to the antibody or antigen-binding fragment thereof.

In certain embodiments, the modification moiety is a detectable label, such as enzyme, radionuclide, fluorescent dye, luminescent substance (e.g., chemiluminescent substance), or biotin. In certain embodiments, the conjugate comprises the antibody or antigen-binding fragment thereof of the present invention and a detectable label attached to the antibody or antigen-binding fragment thereof. The detectable label of the present invention can be any substance detectable by fluorescent, spectroscopic, photochemical, biochemical, immunological, electrical, optical or chemical means. Such labels are well known in the art, examples of which include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin ( PE), Texas red, rhodamine, quantum dots or cyanine derivatives (e.g., Cy7, Alexa 750)), luminescent substances (e.g., chemiluminescent substances such as acridine esters), magnetic beads (e.g., Dynabeads^{®}), calorimetric labels such as colloidal gold or colored glass or plastic beads (e.g., polystyrene, polypropylene, latex, etc.), and biotins to bind an avidin (e.g., streptavidin) modified by the above labels. Patents teaching the use of these markers include, but are not limited to, US Patents 3,817,837; 3,850,752; 3,939,350; 3,996,345; 4,277,437; 4,275,149; and 4,366,241 (all incorporated herein by reference). Detectable labels as described above can be detected by methods known in the art. For example, radiolabels can be detected using photographic film or a scintillation calculator, and fluorescent labels can be detected using a photodetector to detect the emitted light. Enzyme labels are generally detected by providing a substrate to the enzyme and detecting the reaction product produced by the action of the enzyme on the substrate, and calorimetric labels are detected by simply visualizing the colored labels. In certain embodiments, such labels can be suitable for the use in immunological detection (e.g., enzyme-linked immunoassays, radioimmunoassays, fluorescent immunoassays, chemiluminescence immunoassays, etc.). In certain embodiments, a detectable label as described above can be attached to the antibody or antigen-binding fragment thereof of the present invention via a linker of various lengths to reduce potential steric hindrance.

In certain embodiments, the antibody or antigen-binding fragment thereof of the present invention may be linked to a therapeutic moiety. In such embodiments, because the conjugate has the ability to selectively deliver one or more therapeutic agents to a target tissue (e.g. a cell expressing BCMA), the conjugate may enhance the therapeutic efficacy of the antibody or antigen-binding fragment thereof of the present invention in the treatment of a disease (e.g. B cell-related disease).

Thus, in certain embodiments, the modification moiety is a therapeutic agent. In certain embodiments, the conjugate comprises the antibody or antigen-binding fragment thereof of the present invention and a therapeutic agent linked to the antibody or antigen-binding fragment thereof.

In certain embodiments, the conjugate is an antibody-drug conjugate (ADC).

In certain embodiments, the therapeutic agent is a cytotoxic agent. In the present invention, the cytotoxic agent comprises any agent that is detrimental to a cell (e.g., kills a cell).

In certain embodiments, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof.

Examples of alkylating agent that can be used in the conjugate of the present invention include, but are not limited to, nitrogen mustards (e.g., dichloroethylmethylamine, phenylbutyric acid mustard, melphalan, cyclophosphamide, etc.), ethyleneimines (e.g., thiotepae etc.), sulfonates and polyols (e.g., busulfan, dibromomannitol), nitrosoureas (e.g., carmustine, lomustine, etc.), platinum-based antitumor agents (e.g., rdscisplatin, oxaliplatin, carboplatin, etc.).

Examples of mitotic inhibitor that can be used in the conjugate of the present invention include, but are not limited to, maytansinoids (e.g., maytansine, maytansinol, C-3 esters of maytansinol, etc.), taxanes (e.g., docetaxel, paclitaxel or nanoparticle paclitaxel, etc.), vinca rosea alkaloids (e.g., vinblastine sulfate, vincristine, vinblastine, or vinorelbine, etc.).

Examples of antitumor antibiotic that can be used in the conjugate of the present invention include, but are not limited to, actinomycin, anthracycline antibiotics (e.g., daunorubicin, doxorubicin, epirubicin, idarubicin, etc.), calicheamicin, duocarmycin, etc.

Examples of antimetabolite that can be used in the conjugate of the present invention include, but are not limited to, folate antagonists (e.g., methotrexate, etc.), pyrimidine antagonists (e.g., 5-fluorouracil, fluorouridine, cytarabine, capecitabine, gemcitabine, etc.), purine antagonists (e.g., 6-mercaptopurine, 6-thioguanine, etc.), adenosine deaminase inhibitors (e.g., cladribine, fludarabine, nelarabine, pentostatin, etc.).

Examples of topoisomerase inhibitor that can be used in the conjugate of the present invention include, but are not limited to, camptothecins and derivatives thereof (e.g., irinotecan, topotecan, etc.), amsacrine, daunomycin, adriamycin, epipodophyllotoxin, ellipticine, epirubicin, etoposide, razoxane, teniposide, etc.

Examples of tyrosine kinase inhibitor that can be used in the conjugate of the present invention include, but are not limited to, axitinib, bosutinib, sildenib, dasatinib, erlotinib, gefitinib, imatinib, lapatinib, lestaurtinib, nilotinib, semaxanib, sunitinib, vandetanib, etc.

Examples of radionuclide agent that can be used in the conjugate of the present invention include, but are not limited to, I¹³¹, In¹¹¹, Y⁹⁰, Lu¹⁷⁷, and the like.

In certain exemplary embodiments, the therapeutic agent is selected from the group consisting of platinum-based antineoplastic agents, anthracyclines, taxanes, nucleoside analogs, camptothecin compounds, and analogs or homologs thereof, and any combination thereof.

In certain embodiments, the antibody or antigen-binding fragment thereof of the present invention is optionally conjugated to a modification moiety (e.g., a detectable label or therapeutic agent) via a linker.

In the present invention, a cytotoxic agent can be conjugated to the antibody or antigen-binding fragment thereof of the present invention using a linker technology available in the art. Examples of the types of linkers that have been used to conjugate cytotoxic agents to antibodies include, but are not limited to, hydrazones, thioethers, esters, disulfides, and peptide-containing linkers. Linkers that are, for example, susceptible to cleavage by low pH within lysosomal compartment or by proteases (e.g., protease preferentially expressed in tumor tissues, such as cathepsins, such as cathepsins B, C, D) can be selected.

Further discussion of cytotoxic types, linkers, and methods for coupling therapeutic agents to antibodies can be found in Saito, G. et al. (2003) Adv. Drug Deliv. Rev. 55:199-215; Trail, PA et al. (2003) Cancer Immunol. Immunol. 52:328-337; Payne, G. (2003) Cancer Cell 3: 207-212; Allen, TM (2002) Nat. Rev. Cancer 2: 750-763; Pastan, I. and Kreitman, RJ (2002) Curr. Opin Investig. Drugs 3: 1089-1091; Senter, PD and Springer, CJ (2001) Adv. Drug Deliv. Rev. 53: 247-264.

### Chimeric antigen receptor

The antibody or antigen-binding fragment thereof of the present invention can be used to construct a chimeric antigen receptor (CAR). The features of the CAR of the present invention include non-MHC-restricted BCMA recognition ability, which confers on an immune effector cell (e.g., T cell, T cell, NK cell, monocyte, macrophage, or dendritic cell) that expresses the CAR an ability to recognize a BCMA-expressing cell without depending on antigen processing and presentation.

Accordingly, in a sixth aspect, the present invention provides a chimeric antigen receptor (CAR), which comprises an extracellular antigen-binding domain, a spacer domain, a transmembrane domain and an intracellular signaling domain, wherein the extracellular antigen-binding domain comprises the antibody or antigen-binding fragment thereof of the present invention. In certain embodiments, the CAR comprises an extracellular antigen-binding domain, a spacer domain, a transmembrane domain, and an intracellular signaling domain from the N-terminus to the C-terminus.

### 1. Extracellular antigen-binding domain

The extracellular antigen-binding domain contained in the CAR of the present invention confers on the CAR an ability to recognize BCMA.

In certain embodiments, the extracellular antigen-binding domain comprises a heavy chain variable region (VH) and a light chain variable region (VL), the VH comprising a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 2 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto.

In certain embodiments, the extracellular antigen-binding domain comprises a heavy chain variable region (VH) and a light chain variable region (VL), the VH comprising a sequence set forth in SEQ ID NO: 3 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 4 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto.

In certain embodiments, the extracellular antigen-binding domain includes, but is not limited to, Fab fragment, Fab' fragment, F(ab)'₂ fragment, Fv, disulfide-stabilized Fv protein ("dsFv"), scFv, di-scFv, (scFv)₂.

In certain embodiments, the antibody or antigen-binding fragment thereof is a scFv, di-scFv, or (scFv)₂.

In certain embodiments, the extracellular antigen-binding domain further comprises a linker. In certain embodiments, the VH and VL contained in the extracellular antigen-binding domain are linked by a linker. In certain exemplary embodiments, the linker has a sequence set forth in SEQ ID NO:5.

### 2. Transmembrane domain

The transmembrane domain contained in the CAR of the present invention may be any protein structure known in the art, as long as it can be thermodynamically stable in a cell membrane (especially eukaryotic cell membrane). The transmembrane domain suitable for use in the CAR of the present invention may be derived from a natural source. In such embodiments, the transmembrane domain can be derived from any membrane-bound or transmembrane protein. Alternatively, the transmembrane domain may be a synthetic non-naturally occurring protein segment, for example, a protein segment comprising predominantly hydrophobic residues such as leucine and valine.

In certain embodiments, the transmembrane domain is a transmembrane region of a protein selected from the group consisting of α, β or ζ chains of T cell receptor, CD8α, CD28, CD3ε, CD3ζ, CD45, CD4, CD5, CD9, CD16, CD22, CD33, CD37, CD64, CD80, CD86, CD134, CD137, CD154, DAP10, and any combination thereof. In certain exemplary embodiments, the transmembrane domain comprises a transmembrane region of CD8α. In certain exemplary embodiments, the transmembrane domain comprises a transmembrane domain of T cell costimulatory molecule (e.g., CD137 or CD28).

In certain exemplary embodiments, the transmembrane domain comprises an amino acid sequence set forth in SEQ ID NO:7.

### 3. Spacer domain

The chimeric antigen receptor of the present invention may comprise a spacer domain between the extracellular antigen-binding domain and the transmembrane domain.

In certain embodiments, the spacer domain comprises CH2 and CH3 regions of an immunoglobulin (e.g., IgGl or IgG4). In such embodiments, without being bound by a particular theory, it is believed that CH2 and CH3 extend the antigen-binding domain of the CAR from the cell membrane of the CAR-expressing cell and may more accurately mimic the size and domain structure of native TCR.

In certain embodiments, the spacer domain comprises a hinge domain. A hinge domain can be an amino acid segment commonly found between two domains of a protein, which may allow for flexibility of the protein and movement of one or both of the domains relative to one another. Thus, any amino acid sequence that provides such flexibility and movement of the extracellular ligand-binding domain relative to the transmembrane domain can be used for the hinge domain.

In certain embodiments, the hinge domain is a hinge region of a naturally occurring protein or a portion thereof. In certain embodiments, the hinge domain comprises a hinge region of CD8α, or a portion thereof, for example, a fragment comprising at least 15 (e.g., 20, 25, 30, 35 or 40) contiguous amino acids of the hinge region of CD8α. In certain exemplary embodiments, the spacer domain comprises an amino acid sequence set forth in SEQ ID NO:6.

### 4. Intracellular signaling domain

The intracellular signaling domain included in the CAR of the present invention participates in the transduction of the signal of an effective antigen-receptor binding (binding of the CAR of the present invention to BCMA) into an immune effector cell, and activates at least one normal effector function of the CAR-expressing immune effector cell, or enhances the secretion of at least one cytokine (e.g., IL-2, IFN-γ) by the CAR-expressing immune effector cell.

In certain embodiments, the intracellular signaling domain comprises a primary signaling domain and/or a costimulatory signaling domain.

In the present invention, the primary signaling domain may be any intracellular signaling domain comprising an immunoreceptor tyrosine activation motif (ITAM). In certain embodiments, the primary signaling domain comprises an immunoreceptor tyrosine activation motif (ITAM). In certain embodiments, the primary signaling domain comprises an intracellular signaling domain of a protein selected from CD3ζ, FcRy, FcRβ, CD3γ, CD3δ, CD3ε, CD22, CD79a, DAP10, CD79b or CD66d. In certain embodiments, the primary signaling domain comprises an intracellular signaling domain of CD3ζ.

In the present invention, the costimulatory signaling domain may be an intracellular signaling domain from a costimulatory molecule. In certain embodiments, the costimulatory signaling domain comprises an intracellular signaling domain of a protein selected from the group consisting of CARD11, CD2, CD7, CD27, CD28, CD30, CD134 (OX40), CD137 (4-1BB), CD150 (SLAMF1), CD270 (HVEM) or DAP10.

In certain embodiments, the costimulatory signaling domain is selected from an intracellular signaling domain of CD28, or an intracellular signaling domain of CD137(4-1BB), or a combination of fragments of the two.

In certain embodiments, the intracellular signaling domain comprises one costimulatory signaling domain. In certain embodiments, the intracellular signaling domain comprises two or more costimulatory signaling domains. In such embodiments, the two or more costimulatory signaling domains may be the same or different.

In certain embodiments, the intracellular signaling domain comprises a primary signaling domain and at least one costimulatory signaling domain. The primary signaling domain and at least one costimulatory signaling domain can be linked in series to the carboxyl end of the transmembrane domain in any order.

In certain embodiments, the intracellular signaling domain may comprise an intracellular signaling domain of CD3ζ and an intracellular signaling domain of CD137. In certain exemplary embodiments, the intracellular signaling domain of CD3ζ comprises an amino acid sequence set forth in SEQ ID NO:9. In certain exemplary embodiments, the intracellular signaling domain of CD137 comprises an amino acid sequence set forth in SEQ ID NO:8.

In certain embodiments, the CAR of the present invention may further comprise a signal peptide at its N-terminus. Typically, a signal peptide is a polypeptide sequence that targets the sequence to which it is linked to a desired site in a cell. In certain embodiments, the signal peptide can target the CAR to which it is attached to a secretory pathway of the cell and allow the CAR to be further integrated and anchored into a lipid bilayer. Useful signal peptides for CARs are known to those skilled in the art. In certain embodiments, the signal peptide comprises a heavy chain signal peptide (e.g., a heavy chain signal peptide of IgGl), a granulocyte-macrophage colony stimulating factor receptor 2 (GM-CSFR2) signal peptide, or a CD8α signal peptide.

### 5. Full-length CAR

The present invention provides a chimeric antigen receptor capable of specifically binding to BCMA, the chimeric antigen receptor comprises an extracellular antigen-binding domain, a spacer domain, a transmembrane domain, an intracellular signaling domain from the N-terminus to the C-terminus. In certain preferred embodiments, the intracellular signaling domain comprises a costimulatory signaling domain and a primary signaling domain from the N-terminus to the C-terminus.

In certain embodiments, the spacer domain comprises a hinge region of CD8 (e.g., CD8α), which has a sequence set forth in SEQ ID NO:6. In certain embodiments, the transmembrane domain comprises a transmembrane region of CD8 (e.g., CD8α), which has a sequence set forth in SEQ ID NO:7.

In certain embodiments, the intracellular signaling domain comprises a primary signaling domain and a costimulatory signaling domain, wherein the primary signaling domain comprises an intracellular signaling domain of CD3ζ, which has a sequence set forth in SEQ ID NO : 9. The costimulatory signaling domain comprises an intracellular signaling domain of CD137, which has a sequence set forth in SEQ ID NO:8.

In certain exemplary embodiments, the CAR has an amino acid sequence selected from (1) an amino acid sequence set forth in SEQ ID NO: 10 or 12, (2) a sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% compared with the amino acid sequence set forth in SEQ ID NO: 10 or 12, and the sequence substantially retains at least one biological activity of the amino acid sequence from which it is derived (e.g., activity of directing specificity and reactivity of an immune effector cell to a BCMA-expressing cell in a non-MHC restriction manner).

### Preparation of chimeric antigen receptor

Methods of generating chimeric antigen receptors, and immune effector cells (e.g., T cells) comprising the chimeric antigen receptors are known in the art and are described in detail in, for example, Brentjens et al., 2010, Molecular Therapy, 18 :4,666-668; Morgan et al, 2010, Molecular Therapy, published online February 23, 2010, pp. 1-9; Till et al, 2008, Blood, 112:2261-2271; Park et al, Trends Biotechnol., 29 : 550-557, 2011; Grupp et al., NEnglJMed., 368:1509-1518, 2013; Han et al., J.Hematol Oncol., 6:47, 2013; PCT patent publications WO2012/079000, WO2013/126726; and US Patent Publication 2012/0213783, all of which are incorporated herein by reference in their entirety. For example, it may comprise introducing at least one nucleic acid molecule encoding CAR into a cell, and expressing the nucleic acid molecule in the cell. For example, a nucleic acid molecule encoding the CAR of the present invention can be contained in an expression vector (e.g., a lentiviral vector), and the expression vector is capable of being expressed in a host cell, such as a T cell, to manufacture the CAR

Accordingly, in a seventh aspect, the present invention provides an isolated nucleic acid molecule, which comprises a nucleotide sequence encoding the chimeric antigen receptor of the present invention. In certain embodiments, the isolated nucleic acid molecule encodes the chimeric antigen receptor of the present invention.

In certain embodiments, the CAR encoded by the isolated nucleic acid molecule comprises an extracellular antigen-binding domain having a heavy chain variable region (VH) and/or a light chain variable region (VL), wherein: the VH comprises a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprises a sequence set forth in SEQ ID NO: 2 or a sequence having a sequence identity of at least 80%, at least 85%, at least 90%, at least 91%, at least 92%, at least 93%, at least 94%, at least 95%, at least 96%, at least 97%, at least 98%, at least 99% compared to %, or 100% compared thereto.

In certain embodiments, the CAR encoded by the isolated nucleic acid molecule comprises an extracellular antigen-binding domain having a heavy chain variable region (VH) and/or a light chain variable region (VL), wherein: the VH comprises a sequence set forth in SEQ ID NO: 3 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprises a sequence set forth in SEQ ID NO: 4 or a sequence having a sequence identity of at least 80%, at least 85%, at least 90%, at least 91%, at least 92%, at least 93%, at least 94%, at least 95%, at least 96%, at least 97%, at least 98%, at least 99% compared to %, or 100% compared thereto.

In certain exemplary embodiments, the CAR encoded by the isolated nucleic acid molecule has an amino acid sequence selected from: (1) an amino acid sequence set forth in SEQ ID NO: 10 or 12, (2) a sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the amino acid sequences set forth in SEQ ID NO: 10 or 12, and the sequence substantially retains at least one biological activity of the amino acid sequence from which it is derived (e.g., ability of directing specificity and reactivity of an immune effector cell to a BCMA-expressing cell in a non-MHC-restricted manner).

Those skilled in the art understand that, due to the degeneracy of genetic codes, a nucleotide sequence encoding the chimeric antigen receptor of the present invention may have a variety of different sequences. Thus, unless stated otherwise, a "nucleotide sequence encoding an amino acid sequence" includes all nucleotide sequences that are a degenerate form of each other and that encode the same amino acid sequence.

In certain exemplary embodiments, the nucleotide sequence encoding the chimeric antigen receptor of the present invention is selected from: (1) a nucleotide sequence set forth in SEQ ID NO: 11 or 13; (2) a sequence having a sequence identity of at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% compared to the nucleotide sequence set forth in SEQ ID NO: 11 or 13, and the sequence substantially retains at least one biological activity of the nucleotide sequence from which it is derived (e.g., capable of encoding a CAR with the ability of directing specificity and reactivity of an immune effector cell to a BCMA-expressing cell in a non-MHC-restricted manner).

In an eighth aspect, the present invention provides a vector (e.g., cloning vector or expression vector), which comprises the isolated nucleic acid molecule as described above.

In certain embodiments, the vector comprises a nucleotide sequence encoding the chimeric antigen receptor of the present invention.

In certain exemplary embodiments, the CAR has an amino acid sequence selected from: (1) an amino acid sequence set forth in SEQ ID NO: 10 or 12, (2) a sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the amino acid sequences set forth in SEQ ID NO: 10 or 12, and the sequence substantially retains at least one biological activity of the amino acid sequence from which it is derived (e.g., ability of directing specificity and reactivity of an immune effector cell to a BCMA-expressing cell in a non-MHC-restricted manner).

In certain exemplary embodiments, the nucleotide sequence encoding the chimeric antigen receptor of the present invention is selected from: (1) a nucleotide sequence set forth in SEQ ID NO: 11 or 13; (2) a sequence having a sequence identity of at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% compared to the nucleotide sequence set forth in SEQ ID NO: 11 or 13, and the sequence substantially retains at least one biological activity of the nucleotide sequence from which it is derived (e.g., capable of encoding a CAR with the ability of directing specificity and reactivity of an immune effector cell to a BCMA-expressing cell in a non-MHC-restricted manner).

In certain embodiments, the vector is selected from the group consisting of DNA vector, RNA vector, plasmid, transposon vector, CRISPR/Cas9 vector, viral vector.

In certain embodiments, the vector is an expression vector.

In certain embodiments, the vector is an episomal vector.

In certain embodiments, the vector is a viral vector.

In certain exemplary embodiments, the viral vector is a lentiviral, adenoviral, or retroviral vector.

In certain embodiments, the vector is an episomal or non-integration viral vector, such as an integration-deficient retrovirus or lentivirus.

### Modified immune effector cell and preparation method thereof

In a ninth aspect, the present invention provides an immune effector cell expressing the chimeric antigen receptor of the present invention. The CAR can be synthesized in situ in the cell after a polynucleotide encoding the CAR is introduced into the cell by a variety of methods. Alternatively, the CAR can be produced extracellularly and then introduced into the cell.

In certain embodiments, the immune effector cell comprises the isolated nucleic acid molecule of the seventh aspect or the vector of the eighth aspect.

The isolated nucleic acid molecule or vector as described above can be introduced into the immune effector cell by various suitable means, such as calcium phosphate transfection, DEAE-dextran mediated transfection, microinjection, electroporation, TALEN method, ZFN method, non-viral vector-mediated transfection (e.g., liposome) or viral vector-mediated transfection (e.g., lentiviral infection, retroviral infection, adenoviral infection), and other physical, chemical or biological means for transferring it into host cells, such as transposon technology, CRISPR-Cas9 and other technologies.

In certain embodiments, the immune effector cell is derived from a patient or healthy donor. In certain embodiments, the immune effector cell is selected from T lymphocyte, natural killer (NK) cell, monocyte, macrophage or dendritic cell, and any combination thereof. In certain embodiments, the immune effector cell is selected from T lymphocyte and/or natural killer (NK) cell.

In another aspect, the present invention also provides a method for preparing an immune effector cell expressing the chimeric antigen receptor of the present invention, comprising: (1) providing an immune effector cell; (2) introducing the isolated nucleic acid molecule of the sixth aspect or the vector of the seventh aspect into the immune effector cell. The isolated nucleic acid molecule or vector comprises a nucleotide sequence encoding the chimeric antigen receptor of the present invention.

In certain embodiments, the immune effector cell is selected from the group consisting of T lymphocyte, NK cell, monocyte, dendritic cell, macrophage, and any combination thereof.

In certain embodiments, in step (1), the immune effector cell is subjected to a pretreatment; the pretreatment comprises sorting, activation and/or proliferation of the immune effector cell. In certain embodiments, the pretreatment comprises contacting the immune effector cell with an anti-CD3 antibody and an anti-CD28 antibody, thereby stimulating the immune effector cell and inducing its proliferation, thereby generating a pretreated immune effector cell.

In some embodiments, in step (2), the nucleic acid molecule or vector is introduced into the immune effector cell by viral infection. In other embodiments, in step (2), the nucleic acid molecule or vector is introduced into the immune effector cell by means of non-viral vector transfection, such as vector system by transposon, CRISPR/Cas9 vector, TALEN method, ZFN method, electroporation method, calcium phosphate transfection, DEAE-dextran mediated transfection or microinjection method.

In certain embodiments, after step (2), the method further comprises: expanding the immune effector cell obtained in step (2).

In another aspect, the present invention also provides a kit for preparing a chimeric antigen receptor capable of specifically binding to BCMA or a cell expressing the chimeric antigen receptor, the kit comprising the isolated nucleic acid molecule of the seventh aspect, or the vector of the eighth aspect, and an necessary solvent (for example, sterile water or physiological saline, or cell culture medium). The kit optionally comprises an instruction for use.

In another aspect, the present invention provides a use of the above kit in the manufacture of a chimeric antigen receptor capable of specifically binding to BCMA or a cell expressing the chimeric antigen receptor.

### Pharmaceutical composition

In a tenth aspect, the present invention provides a pharmaceutical composition, which comprises the antibody or antigen-binding fragment thereof of the first aspect of the present invention, the conjugate of the fifth aspect, the chimeric antigen receptor of the sixth aspect, the isolated nucleic acid molecule of the second aspect or the seventh aspect, the vector of the third aspect or the eighth aspect, the host cell of the fourth aspect or the immune effector cell of the ninth aspect, and a pharmaceutically acceptable carrier and/or excipient.

In some embodiments, the pharmaceutical composition of the present invention comprises the antibody or antigen-binding fragment thereof of the present invention.

In some embodiments, the pharmaceutical composition of the present invention comprises the conjugate of the present invention. In such embodiments, the conjugate comprises the antibody or antigen-binding fragment thereof of the present invention and a therapeutic agent linked to the antibody or antigen-binding fragment thereof.

In some embodiments, the pharmaceutical composition of the present invention comprises the isolated nucleic acid molecule of the seventh aspect, the vector of the eighth aspect, or the immune effector cell of the ninth aspect. The nucleic acid molecule and vector comprise a nucleotide sequence encoding the chimeric antigen receptor of the present invention, and the immune effector cell expresses the chimeric antigen receptor.

In certain embodiments, the pharmaceutical composition may further comprise an additional pharmaceutically active agent.

In certain embodiments, the additional pharmaceutically active agent is a drug with antitumor activity, for example, alkylating agent, mitotic inhibitor, antitumor antibiotic, antimetabolite, topoisomerase inhibitor, tyrosine kinase inhibitor, radionuclide agent, radiosensitizer (e.g., gemcitabine, 5-fluorouracil, taxane, cisplatin, etc.), anti-angiogenic agent, cytokine (e.g., GM-CSF, IL-7, IL-12, IL-15, IL-18, IL-21, etc.), antibody specifically targeting tumor cell (e.g., CD20 antibody such as rituximab, Her2 antibody such as trastuzumab, VEGF antibody such as bevacizumab, EGFR antibody such as cetuximab, etc.), immune checkpoint inhibitor (e.g., PD-1 antibody, PD-L1 antibody, CTLA-4 antibody, LAG-3 antibody, etc.), and so on.

In certain embodiments, in the pharmaceutical composition, the antibody or antigen-binding fragment thereof, conjugate, isolated nucleic acid molecule, vector or immune effector cell of the present invention and the additional pharmaceutically active agent of the present invention may be provided as separated components or mixed components. Thus, the antibody or antigen-binding fragment, conjugate, isolated nucleic acid molecule, vector or immune effector cell of the present invention and the additional pharmaceutically active agent can be administered simultaneously, separately or sequentially.

The antibody or antigen-binding fragment thereof, conjugate, immune effector cell or pharmaceutical composition of the present invention can be formulated into any dosage form known in the medical art, for example, tablet, pill, suspension, emulsion, solution, gel, capsule, powder, granule, elixir, lozenge, suppository, injection (including injection, sterile powder for injection and concentrated solution for injection), inhalant, spray, etc. The preferred dosage form depends on the intended mode of administration and therapeutic use. The pharmaceutical composition of the present invention should be sterile and stable under the conditions of manufacture and storage. A preferred dosage form is an injection. Such injection can be a sterile injectable solution. For example, the sterile injectable solution can be prepared by the following method: incorporating in an appropriate solvent a necessary dose of the antibody or antigen-binding fragment thereof, conjugate, immune effector cell, or pharmaceutical composition of the present invention, and optionally incorporating other desired ingredients (including, but not limited to, pH adjuster, surfactant, adjuvant, ionic strength enhancer, isotonic agent, preservative, diluent, or any combination thereof), followed by filtration and sterilization. In addition, the sterile injectable solution can be prepared as a sterile lyophilized powder (e.g., by vacuum drying or freeze-drying) for ease storage and use. Such sterile lyophilized powder can be dispersed in a suitable vehicle such as water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffered solution (e.g., phosphate buffered solution), Ringer's solution, and any combination thereof.

Accordingly, in certain exemplary embodiments, the pharmaceutical compositions of the present invention comprise sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffered solution (e.g., phosphate buffered solution), Ringer's solution, and any combination thereof.

The antibody or antigen-binding fragment thereof, conjugate, immune effector cell, or pharmaceutical composition of the present invention can be administered by any suitable method known in the art, including, but not limited to, oral, buccal, sublingual, ocular, local, parenteral, rectal, intrathecal, intracytoplasmic reticulum, inguinal, intravesical, topical (e.g., powder, ointment, or drop), or nasal route. However, for many therapeutic uses, the preferred route/mode of administration is parenteral (e.g., intravenous or bolus injection, subcutaneous injection, intraperitoneal injection, intramuscular injection). The skilled in the art will appreciate that the route and/or mode of administration will vary depending on the intended purpose. In certain embodiments, the antibody or antigen-binding fragment thereof, conjugate, immune effector cell, or pharmaceutical composition of the present invention is administered by intravenous injection or bolus injection.

The pharmaceutical composition of the present invention may comprise a "therapeutically effective amount" or a "prophylactically effective amount" of the antibody or antigen-binding fragment thereof described in the first aspect, the conjugate described in the fifth aspect, or the immune effector cell described in the eighth aspect of the present invention. The "prophylactically effective amount" refers to an amount sufficient to cure, prevent, or delay the onset of a disease. The "therapeutically effective amount" refers to an amount sufficient to cure or at least partially prevent a disease and its complications in a patient already suffering from the disease. The therapeutically effective amount of the antibody or antigen-binding fragment thereof of the present invention, the conjugate of the fifth aspect, or the immune effector cell of the eighth aspect may vary according to the following factors: the severity of the disease to be treated, the general state of the patient's own immune system, the patient's general conditions such as age, weight and sex, the mode of administration, and other therapies simultaneously administered, etc.

### Therapeutic methods and uses

In another aspect, the present invention provides a method for preventing and/or treating a B cell-related disease in a subject (e.g., human), the method comprising administering to the subject in need thereof an effective amount of the antibody or antigen-binding fragment thereof of the first aspect, the conjugate of the fifth aspect, the immune effector cell of the eighth aspect, or the pharmaceutical composition of the present invention. In such embodiments, the conjugate comprises the antibody or antigen-binding fragment thereof of the present invention and a therapeutic agent linked to the antibody or antigen-binding fragment thereof.

In certain embodiments, the methods comprise administering to the subject an effective amount of the antibody or antigen-binding fragment thereof or conjugate of the present invention.

In certain embodiments, the method comprises administering to the subject an effective amount of the immune effector cell of the eighth aspect or the pharmaceutical composition comprising the immune effector cell.

In certain embodiments, the method comprises the following steps: (1) providing an immune effector cell (e.g., T lymphocyte, NK cell, monocyte, macrophage, dendritic cell, or any combination of these cells); (2) introducing a nucleic acid molecule encoding the chimeric antigen receptor described in the sixth aspect of the present invention into the immune effector cell described in step (1) to obtain an immune effector cell expressing the chimeric antigen receptor; (3) administering the immune effector cell obtained in step (2) to the subject for treatment.

In certain embodiments, prior to step (1), the method comprises a step of obtaining the immune effector cell from the subject. In certain embodiments, the immune effector cell is selected from T lymphocyte and/or NK cell.

In certain exemplary embodiments, a peripheral blood mononuclear cell (PBMC) is obtained from the subject, and the PBMC is directly genetically modified to express the CAR

In certain exemplary embodiments, the T cell is obtained from the subject. The T cell can be obtained from a variety of sources including, but not limited to, peripheral blood mononuclear cell, bone marrow, lymph node tissue, umbilical cord blood, thymus tissue, tissue from infection site, ascites, pleural effusion, spleen tissue, and tumor. In certain embodiments, the T cell can be obtained from blood units collected from a subject using a variety of techniques known to the skilled artisan (e.g., deposition, for example, FICOLL^{™} isolation). In one embodiment, the cells from the circulating blood of an individual are obtained by apheresis. The product of apheresis usually contains lymphocytes, including T cells, monocytes, granulocytes, B cells, other nucleated leukocytes, red blood cells, and platelets. In one embodiment, the cells collected by apheresis can be washed to remove plasma fractions and placed in a suitable buffer or medium for subsequent processing. The Cells can be washed with PBS or other suitable solution devoid of calcium, magnesium and most of divalent cations (if not all other divalent cations). The washing step can be accomplished by methods known to those of ordinary skill in the art, such as by using a semiautomatic flow-through centrifuge, as understood by those of ordinary skill in the art. Examples include Cobe 2991 Cell Processor, Baxter CytoMate, etc. After washing, the cells can be resuspended in various biocompatible buffers or other saline solutions with or without buffers. In certain embodiments, unwanted components of apheresis samples can be removed from the medium in which the cells are directly resuspended.

In certain embodiments, the T cell is isolated from peripheral blood mononuclear cells (PBMCs) by lysing red blood cells and depleting monocytes (e.g., by centrifugation through a PERCOLL^{™} gradient). A specific subpopulation of T cells expressing one or more of the following markers: CD3, CD28, CD4, CD8, CD45RA and CD45RO, can be further isolated by positive or negative selection techniques. In one embodiment, a specific subpopulation of T cells expressing CD3, CD28, CD4, CD8, CD45RA and CD45RO are further isolated by positive or negative selection techniques. For example, enrichment of a T cell population by negative selection may be accomplished with a combination of antibodies directed to surface markers unique to the negatively selected cells. An exemplary method is cell sorting and/or selection via negative magnetic immunoadherence or flow cytometry, in which the negative magnetic immunoadhesion or flow cytometry utilizes a mixture of monoclonal antibodies directed to cell surface markers present on the cells negatively selected. For example, in order to enrich CD4+ cells by negative selection, a mixture of monoclonal antibodies typically contain antibodies against CD14, CD20, CD11b, CD16, HLA-DR, and CD8. Flow cytometry and cell sorting can also be used to isolate cell populations of interest for the use in the present invention.

After isolation, the immune effector cell (e.g., T cell) is genetically modified, or the immune effector cell can be activated and expanded (or, in the case of progenitor cell, differentiated) in vitro prior to the genetic modification. In certain exemplary embodiments, the immune effector cell (e.g., T cell) is genetically modified (e.g., transduced with a viral vector comprising a nucleic acid encoding the CAR) with the chimeric antigen receptor of the present invention, and then subjected to in vitro activation and expansion.

In certain embodiments, the B cell-related disease is a B cell malignancy, such as multiple myeloma (MM) or non-Hodgkin's lymphoma (NHL). In certain embodiments, the multiple myeloma (MM) is selected from the group consisting of: overt multiple myeloma, smoldering multiple myeloma, plasma cell leukemia, nonsecretory myeloma, IgD myeloma, osteosclerotic myeloma, solitary bone plasmacytoma, and extramedullary plasmacytoma. In certain embodiments, the non-Hodgkin's lymphoma (NHL) is selected from: Burkitt's lymphoma, chronic lymphocytic leukemia/small lymphocytic lymphoma (CLL/SLL), diffuse large B-cell lymphoma tumor, follicular lymphoma, immunoblastic large cell lymphoma, precursor B lymphoblastic lymphoma, and mantle cell lymphoma.

In certain embodiments, the B cell-related disease is a plasma cell malignancy.

In certain embodiments, the B cell-related disease is an autoimmune disease. In certain embodiments, the autoimmune disease is selected from the group consisting of: systemic lupus erythematosus, rheumatoid arthritis, idiopathic thrombocytopenic purpura or myasthenia gravis or autoimmune hemolytic anemia.

In certain embodiments, the B cell-related disease is selected from the group consisting of multiple myeloma, non-Hodgkin's lymphoma, B cell proliferation of uncertain malignant potential, lymphomatoid granulomatosis, post-transplant lymphoproliferative disease, immunomodulatory disorder, rheumatoid arthritis, myasthenia gravis, idiopathic thrombocytopenic purpura, antiphospholipid syndrome, Chagas' disease, Graves' disease, Wegener's granulomatosis, polyarteritis nodosa, Sjogren's syndrome, pemphigus vulgaris, scleroderma, multiple sclerosis, antiphospholipid syndrome, ANCA-associated small vessel vasculitis, Goodpasture's disease, Kawasaki disease, autoimmune hemolysis anemia and rapidly progressive glomerulonephritis, heavy chain disease, primary or immune cell-associated amyloidosis, or monoclonal gammopathy of undetermined significance.

In certain embodiments, the antibody or antigen-binding fragment thereof, conjugate, immune effector cell, or pharmaceutical composition of the present invention is administered in combination with an additional therapy. The additional therapy can be any therapy known for treatment of tumors such as surgery, chemotherapy, radiation therapy, targeted therapy, immunotherapy, hormone therapy, gene therapy, or palliative care. Such additional therapy may be administered prior to, concurrently with, or subsequent to the administration of the antibody or antigen-binding fragment thereof, conjugate, immune effector cell, or pharmaceutical composition of the present invention.

In certain embodiments, the subject may be a mammal, such as a human.

In another aspect, there is provided a use of the antibody or antigen-binding fragment thereof of the first aspect, the conjugate of the fifth aspect, the chimeric antigen receptor of the sixth aspect, the isolated nucleic acid molecule of the second aspect or the seventh aspect, the vector of the third aspect or the eighth aspect, the host cell of the fourth aspect, the immune effector cell of the ninth aspect, or the pharmaceutical composition of the present invention in the manufacture of a medicament, wherein the medicament is used for the prevention and/or treatment of a B cell-related disease in a subject (e.g., a human). In such embodiments, the conjugate comprises the antibody or antigen-binding fragment thereof of the present invention and a therapeutic agent linked to the antibody or antigen-binding fragment thereof.

### Detection method and kit

The antibody or antigen-binding fragment thereof of the present invention can specifically bind to BCMA, and thus can be used to detect the presence or level of BCMA in a sample.

Accordingly, in another aspect, the present invention provides a kit, which comprises the antibody or antigen-binding fragment thereof of the present invention.

In certain embodiments, the kit is used to diagnose whether a subject has a BCMA-expressing tumor. In certain embodiments, the BCMA-expressing tumor is selected from B-cell malignancies such as multiple myeloma (MM) or non-Hodgkin's lymphoma (NHL). In certain embodiments, the BCMA-expressing tumor is a plasma cell malignancy.

In certain preferred embodiments, the antibody or antigen-binding fragment thereof of the present invention bears a detectable label.

In certain preferred embodiments, the kit further comprises a second antibody that specifically recognizes the antibody or antigen-binding fragment thereof of the present invention. Preferably, the second antibody further comprises a detectable label.

In the present invention, the detectable label may be any substance detectable by fluorescent, spectroscopic, photochemical, biochemical, immunological, electrical, optical or chemical means. It is particularly preferred that such label is suitable for the use in immunological detection (e.g., enzyme-linked immunoassay, radioimmunoassay, fluorescent immunoassay, chemiluminescence immunoassay, etc.).

In another aspect, the present invention provides a method for detecting the presence or amount of BCMA in a sample, comprising the steps of:
(1) contacting the sample with the antibody or antigen-binding fragment thereof of the present invention;
(2) detecting the formation of a complex between the antibody or antigen-binding fragment thereof and BCMA or detecting the amount of the complex.

The formation of the complex indicates the presence of BCMA or cell expressing BCMA.

In certain embodiments, the sample is a cellular sample, that is, a sample comprising a cell (e.g., tumor cell). In such embodiments, the complex is preferably formed between the antibody or antigen-binding fragment thereof, or conjugate and the BCMA expressed by cells in the sample.

In certain embodiments, the antibody or antigen-binding fragment thereof of the present invention further bears a detectable label. In certain embodiments, in step (2), the antibody or antigen-binding fragment thereof of the present invention is detected with a reagent bearing a detectable label.

The method may be used for a diagnostic purpose, or for a non-diagnostic purpose (e.g., the sample is a cell sample rather than a sample from a patient). In certain embodiments, the BCMA is human BCMA.

In another aspect, there is provided a use of the antibody or antigen-binding fragment thereof of the present invention in the manufacture of a kit, in which the kit is used for detecting the presence or amount of BCMA in a sample. In certain embodiments, the BCMA is human BCMA.

In another aspect, the present invention provides a method for diagnosing whether a subject has a BCMA-expressing tumor, comprising detecting an amount of BCMA in a sample form the subject by using the antibody or antigen-binding fragment thereof of the first aspect of the present invention.

In certain embodiments, the BCMA-expressing tumor is selected from B cell malignancies, such as multiple myeloma (MM) or non-Hodgkin's lymphoma (NHL). In certain embodiments, the BCMA-expressing tumor is a plasma cell malignancy.

In certain embodiments, the method further comprises a step of comparing the amount of BCMA in the sample from the subject to a reference value.

The reference value refers to the level of BCMA in a sample from a subject known not to have a BCMA-expressing tumor (also referred to as a "negative reference value"), or to the level of BCMA in a sample form a subject known to have a BCMA-expressing tumor (also referred to as a "positive reference value"). For example, if the amount of BCMA in a sample from the subject is similar (or not significantly different) from a negative reference value, it is indicative that the subject does not have a BCMA-expressing tumor, and if the amount of BCMA in a sample from the subject is increased relative to a negative reference value, it is indicative that the subject has a BCMA-expressing tumor. Furthermore, if the amount of BCMA in a sample from the subject is similar (or not significantly different) from a positive reference value, it is indicative that the subject has a BCMA-expressing tumor.

In certain embodiments, the amount of BCMA in a sample from the subject is detected by the steps of:
(1) contacting the sample from the subject with the antibody or antigen-binding fragment thereof of the present invention;
(2) detecting the amount of a complex formed with the antibody or its antigen-binding fragment thereof and BCMA.

In certain embodiments, in step (1), the antibody or antigen-binding fragment thereof of the present invention further bears a detectable label. In certain embodiments, in step (2), the antibody or antigen-binding fragment thereof of the present invention is detected using a reagent bearing a detectable label.

In certain embodiments, the sample may be selected from the group consisting of urine, blood, serum, plasma, saliva, ascites, circulating cell, circulating tumor cell, non-tissue-associated cell (i.e., free cell), tissue (e.g., surgically resected tumor tissue, biopsy or fine needle aspiration tissue), histological preparation, etc.

In certain embodiments, the method further comprises administering to a subject diagnosed with a BCMA-expressing tumor a BCMA-targeted immunotherapy.

In certain embodiments, the BCMA-targeted immunotherapy comprises administering the antibody or antigen-binding fragment thereof, or conjugate of the present invention.

In certain embodiments, the BCMA-targeted immunotherapy comprises administrating the immune effector cell of the present invention.

In certain embodiments, the BCMA-targeted immunotherapy comprises administrating the immune effector cell expressing the chimeric antigen receptor of the sixth aspect of the present invention. The method comprises the following steps: (1) providing an immune effector cell; (2) introducing a nucleotide sequence encoding the chimeric antigen receptor of the present invention into the immune effector cell described in step (1) to obtain the immune effector cell expressing the chimeric antigen receptor; (3) administrating the immune effector cell obtained in step (2) and optionally unengineered and/or unsuccessfully engineered immune effector cells to the subject. In certain embodiments, prior to step (1), the method further comprises a step of obtaining the immune effector cell from the subject.

In another aspect, there is provided a use of the antibody or antigen-binding fragment thereof of the present invention in the manufacture of a kit, in which the kit is used for diagnosing whether a subject has a BCMA-expressing tumor or for detecting whether a tumor can be treated by a BCMA-targeted anti-tumor therapy.

### Definition of terms

In the present invention, unless otherwise specified, scientific and technical terms used herein have the meanings commonly understood by those skilled in the art. Moreover, the operation steps of cell culture, molecular biology, biochemistry, nucleic acid chemistry, immunology and the like used herein are all routine steps widely used in the corresponding fields. Meanwhile, for a better understanding of the present invention, definitions and explanations of related terms are provided below.

As used herein, the term "BCMA" refers to B-cell maturation antigen, also known as TNFRSF17 or CD269. BCMA, a member of the tumor necrosis factor receptor family (TNFR), binds to the TNF family ligands BAFF (B-cell activating factor) and APRIL (proliferation-inducing ligand), and is predominantly expressed in terminally differentiated B cells, such as memory B cells and plasma cells. The gene for BCMA is encoded on chromosome 16, producing a primary mRNA transcript of 994 nucleotides in length (NCBI Accession No. NM_001192.2), which encodes a protein of 184 amino acids (NP_001183.2).

As used herein, the term "antibody" refers to an immunoglobulin molecule that typically consists of two pairs of polypeptide chains, and each pair has a light chain (LC) and a heavy chain (HC). Antibody light chains can be classified as κ (kappa) and λ (lambda) light chains. Heavy chains can be classified as µ, δ, γ, α, or ε, and the isotypes of antibody are defined as IgM, IgD, IgG, IgA, and IgE, respectively. Within the light and heavy chains, the variable and constant regions are linked by a "J" region of about 12 or more amino acids, and the heavy chains further contain a "D" region of about 3 or more amino acids. Each heavy chain is composed of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of a domain CL. The constant domains do not directly participate in the binding of antibody to antigen, but exhibit a variety of effector functions, such as mediating the binding of immunoglobulin to a host tissue or factor, including various cells of the immune system (e.g., effector cells) and the first component (C1q) of classical complement system. The VH and VL regions can also be subdivided into regions with high denaturation (referred to as complementary determining regions (CDRs)), which are interspersed with interspaced conservative regions called framework regions (FRs). Each of V_{H} and V_{L} is composed of 3 CDRs and 4 FRs arranged in the following order: FR1, CDR1, FR2, CDR2, FR3, CDR3 and FR4 from the amino terminal to the carboxy terminal. The variable regions (VH and VL) of each heavy/light chain pair form an antigen-binding site, respectively. The assignment of amino acids in various regions or domains may follow the definitions of Kabat, Sequences of Proteins of Immunological Interest (National Institutes of Health, Bethesda, Md. (1987 and 1991)), or Chothia & Lesk (1987) J. Mol. Biol. 196 : 901-917; Chothia et al. (1989) Nature 342: 878-883.

As used herein, the term "complementary determining region" or "CDR" refers to the amino acid residues in variable region of antibody that are responsible for antigen binding. The variable regions of the heavy and light chains each contain 3 CDRs, named CDR1, CDR2 and CDR3. The precise boundaries of these CDRs can be defined according to various numbering systems known in the art, for example, according to the Kabat numbering system (Kabat et al., Sequences of Proteins of Immunological Interest, 5th Ed. Public Health Service, National Institutes of Health, Bethesda, Md., 1991), Chothia numbering system (Chothia & Lesk (1987) J. Mol. Biol. 196: 901-917; Chothia et al. (1989) Nature 342: 878-883) or IMGT numbering system (Lefranc et al., Dev. Comparat. Immunol. 27: 55-77, 2003). For a given antibody, those skilled in the art will readily identify the CDRs defined by each numbering system. And, the correspondence between different numbering systems is well known to those skilled in the art (e.g., see Lefranc et al., Dev. Comparat. Immunol. 27: 55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia or IMGT numbering system. In certain embodiments, the CDRs contained in the antibody or antigen-binding fragment thereof of the present invention are preferably determined by the Kabat numbering system.

As used herein, the term "framework region" or "FR" residue refers to those amino acid residues other than the CDR residues as defined above in the variable regions of antibody.

The term "antibody" is not limited by any particular method for producing antibody. For example, it includes recombinant antibody, monoclonal antibody and polyclonal antibody. The antibody may be an antibody of different isotypes, for example, IgG (e.g., IgG1, IgG2, IgG3 or IgG4 subtypes), IgA1, IgA2, IgD, IgE or IgM antibody.

As used herein, the term "antigen-binding fragment" of an antibody refers to a polypeptide comprising a fragment of a full-length antibody that retains the ability to specifically bind to the same antigen to which the full-length antibody binds, and/or compete with the full-length antibody to specifically bind to the antigen, which is also referred to as an "antigen binding portion". See generally, Fundamental Immunology, Ch. 7 (Paul, W., ed., 2nd ed., Raven Press, NY (1989), which is hereby incorporated by reference in its entirety for all purposes. Antigen-binding fragments of an antibody can be produced by recombinant DNA techniques or by enzymatic or chemical cleavage of intact antibody. Non-limiting examples of antigen-binding fragments include Fab, Fab', (Fab')₂, Fv, disulfide-linked Fv, scFv, di-scFv, (scFv)₂, and polypeptides comprising at least at least a portion of antibody that is sufficient to confer the specific antigen binding ability to the polypeptide. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains". The "full-length heavy chain" refers to a polypeptide chain consisting of a heavy chain variable region (VH), a heavy chain constant region CH1 domain, a hinge region (HR), a heavy chain constant region CH2 domain and a heavy chain constant region CH3 domain in a direction from the N-terminus to the C-terminus; and, when the full-length antibody is an IgE isotype, the heavy chain constant region CH4 domain is optionally further included. Preferably, the "full-length heavy chain" is a polypeptide chain consisting of VH, CHI, HR, CH2 and CH3 in a direction from the N-terminal to C-terminal. The "full-length light chain" is a polypeptide chain consisting of a light chain variable region (VL) and a light chain constant region (CL) in a direction from the N-terminal to C-terminal. Two pairs of full-length antibody chains are linked together by a disulfide bond between CL and CH1 and a disulfide bond between HRs of two full-length heavy chains. The full-length antibody of the present invention may be from a single species, such as a human; or may be a chimeric or humanized antibody. The full-length antibody of the present invention comprises two antigen-binding sites formed by VH and VL pairs, respectively, and these two antigen-binding sites specifically recognize/bind the same antigen.

As used herein, the term "Fd" refers to an antibody fragment consisting of VH and CH1 domains; the term "dAb fragment" refers to an antibody fragment consisting of VH domain (Ward et al., Nature 341: 544 546 ( 1989)); the term "Fab fragment" refers to an antibody fragment consisting of VL, VH, CL and CH1 domains; the term "F(ab')₂ fragment" refers to an antibody fragment comprising two Fab fragments connected by a disulfide bridge on hinge region; the term "Fab' fragment" refers to a fragment obtained by reducing the disulfide bond connecting the two heavy chain fragments in the F(ab')₂ fragment, which consists of one complete light chain and Fd Fragment (composed of VH and CH1 domains) of heavy chain.

As used herein, the term "Fv" refers to an antibody fragment consisting of VL and VH domains of one arm of antibody. Fv fragment is generally considered to be the smallest antibody fragment that can form a complete antigen-binding site. It is generally believed that 6 CDRs confer antigen-binding specificity to an antibody. However, even a variable region (e.g., an Fd fragment containing only 3 antigen-specific CDRs) is able to recognize and bind an antigen, although its affinity may be lower than that of the complete binding site.

As used herein, the term "Fc" refers to an antibody fragment that is formed by linking the second and third constant regions of the first heavy chain of an antibody with the second and third constant regions of the second heavy chain via a disulfide bond. The Fc fragment of antibody has many different functions, but does not participate in antigen binding.

As used herein, the term "scFv" refers to a single polypeptide chain comprising VL and VH domains, wherein the VL and VH are connected by a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Eds. Roseburg and Moore, Springer-Verlag, New York, pp. 269-315 (1994)). Such scFv molecules can have the general structure: NH₂-VL-linker-VH-COOH or NH₂-VH-linker-VL-COOH. Suitable prior art linkers consist of repeated GGGGS amino acid sequences or variants thereof. For example, the used linker can be the amino acid sequence (GGGGS)₄, but also variants thereof (Holliger et al. (1993), Proc. Natl. Acad. Sci. USA 90: 6444-6448). Other linkers useful in the present invention are described by Alfthan et al. (1995), Protein Eng. 8:725-731, Choi et al. (2001), Eur. J. Immunol. 31:94-106, Hu et al. (1996), Cancer Res. 56:3055-3061, described by Kipriyanov et al. (1999), J. Mol. Biol. 293:41-56 and Roovers et al. (2001), Cancer Immunol. In some cases, a disulfide bond may also exist between the VH and VL of the scFv. In certain embodiments of the present invention, the scFv can form a di-scFv, which refers to an antibody formed with two or more single scFvs connected in series. In certain embodiments of the present invention, the scFv can form (scFv)₂, which refers to an antibody formed with two or more single scFvs connected in parallel.

Each of the aforementioned antibody fragments retains the ability to specifically bind to the same antigen to which the full-length antibody bind, and/or compete with the full-length antibody to specifically bind to the antigen.

Antigen-binding fragments of antibodies (e.g., antibody fragments described above) can be obtained from a given antibody (e.g., the antibody provided in the present invention) using conventional techniques known to those of skill in the art (e.g., recombinant DNA techniques or enzymatic or chemical fragmentation methods), and the antigen-binding fragments of antibodies can be screened for specificity in the same manner as used for intact antibodies.

Herein, unless specified definitely, when referring to the term "antibody", it includes not only the intact antibody but also an antigen-binding fragment of the antibody.

As used herein, the term "humanized antibody" refers to a genetically engineered non-human antibody of which the amino acid sequence is modified to increase homology to the sequence of human antibody. Generally, all or part of CDR regions of humanized antibody is derived from a non-human antibody (donor antibody), and all or part of non-CDR regions (e.g., variable region FR and/or constant region) is derived from a human immunoglobulin (receptor antibody). Humanized antibody generally retains the expected properties of the donor antibody, including, but not limited to, antigen specificity, affinity, reactivity, and the like. The donor antibody can be an antibody of mouse, rat, rabbit, or non-human primate (e.g., cynomolgus monkey) with the expected properties (e.g., antigen specificity, affinity, reactivity, etc.).

Humanized antibodies not only can retain the expected properties of non-human donor antibody (e.g., murine antibody), but also can effectively reduce the immunogenicity of non-human donor antibody (e.g., murine antibody) in a human subject, and thus it is particularly advantageous. However, due to matching issues between the CDRs of donor antibody and the FRs of recipient antibody, the expected properties of humanized antibodies (e.g., antigen specificity, affinity, reactivity, ability to increase immune cell activity, and/or ability to enhance immune response) are generally lower than those of non-human donor antibodies (e.g., murine antibodies).

Therefore, although researchers in the field have carried out in-depth research on the humanization of antibodies and made some progress, there is still lack of a detailed guidance in the art how to fully humanize a certain donor antibody, so that the generated humanized antibody has the highest possible degree of humanization, and can retain the expected properties of the donor antibody as much as possible. Technicians have to explore, research and modify a specific donor antibody by a lot of creative work, to obtain a humanized antibody that has a high degree of humanization and retains the expected properties of the specific donor antibody.

As used herein, the term "germline antibody gene" or "germline antibody gene segment" refers to a sequence present in the genome of an organism encoding immunoglobulin, which has not undergone a maturation process that can lead to genetic rearrangements and mutations for expression of a particular immunoglobulin. In the present invention, the term "heavy chain germline gene" refers to a germline antibody gene or gene fragment encoding an immunoglobulin heavy chain, which comprises a V gene (variable), a D gene (diversity), a J gene (joining) and a C gene (constant); similarly, the term "light chain germline gene" refers to a germline antibody gene or gene fragment encoding an immunoglobulin light chain, which comprises a V gene (variable), a J gene (joining) and a C gene (constant). In the present invention, the amino acid sequence encoded by the germline antibody gene or germline antibody gene fragment is also referred to as a "germline sequence". The germline antibody gene or germline antibody gene fragment as well as corresponding germline sequence thereof are well known to those skilled in the art and can be obtained or queried from professional databases (e.g., IMGT, UNSWIg, NCBI, or VBASE2).

As used herein, the term "specifically bind" or "specific binding" refers to a non-random binding reaction between two molecules, such as a reaction between an antibody and an antigen to which it is directed. The strength or affinity of a specific binding interaction can be expressed in term of the equilibrium dissociation constant (K_{D}) of the interaction. In the present invention, the term "K_{D}" refers to the dissociation equilibrium constant of specific antibody-antigen interaction, which is used to describe the binding affinity between antibody and antigen. The lower the equilibrium dissociation constant, the tighter the antibody-antigen binding, and the higher the affinity between antibody and antigen.

The specific binding properties between two molecules can be determined using methods known in the art. One method involves measuring the formation and dissociation rates of antigen-binding site/antigen complex. Both the "association rate constant" (ka or kon) and the "dissociation rate constant" (kdis or koff) can be calculated from the concentration and the actual association and dissociation rates (see: Malmqvist M, Nature, 1993, 361: 186-187). The ratio of kdis/kon is equal to the dissociation constant K_{D} (see: Davies et al., Annual Rev Biochem, 1990; 59: 439-473). Any effective method can be used to measure K_{D}, kon and kdis values. In certain embodiments, surface plasmon resonance (SPR) can be used to measure the dissociation constant in Biacore. In addition, bioluminescence interferometry or Kinexa can also be used to measure the dissociation constant.

As used herein, the term "chimeric antigen receptor (CAR)" refers to a recombinant polypeptide construct comprising at least one extracellular antigen-binding domain, a spacer domain, a transmembrane domain, and an intracellular signaling domain, which combines the antibody-based specificity for an antigen of interest (e.g., BCMA) with an immune effector cell activating intracellular domain to exhibit specific immune activity against a cell expressing the antigen of interest (e.g., BCMA). In the present invention, the expression "CAR-expressing immune effector cell" refers to an immune effector cell that expresses CAR and has antigen specificity determined by the targeting domain of the CAR. Methods of making CARs (e.g., for cancer therapy) are known in the art, see, for example, Park et al., Trends Biotechnol., 29:550-557, 2011; Grupp et al., NEnglJMed., 368:1509-1518, 2013; Hanetal., J. Hematol Oncol., 6:47, 2013; PCT Patent Publications WO2012/079000, WO2013/059593; and US Patent Publication 2012/0213783, all of which are incorporated herein by reference in their entirety. In this article, the expression "anti-BCMA CAR" refers to a CAR that contains an extracellular antigen-binding domain capable of specifically binding to BCMA; the expression "anti-BCMA CAR-T" refers to an immune effector cell (e.g., PBMC, e.g., T cell) expressing the CAR. As used herein, the expression "humanized CAR" refers to a CAR comprising an extracellular antigen-binding domain derived from a humanized antibody; similarly, the expression "murine CAR" refers to a CAR comprising an extracellular antigen-binding domain derived from a murine antibody.

As used herein, the term "extracellular antigen-binding domain" refers to a polypeptide capable of specifically binding to an antigen or receptor of interest. This domain will be able to interact with a cell surface molecule. For example, the extracellular antigen-binding domain can be selected to recognize an antigen that is a cell surface marker of a target cell associated with a particular disease state. Typically, the extracellular antigen-binding domain is an antibody-derived targeting domain.

As used herein, the term "intracellular signaling domain" refers to a portion of protein that transduces the effector function signal and directs the cell to perform a specialized function. Thus, the intracellular signaling domain has the ability to activate at least one normal effector function of a CAR-expressing immune effector cell. For example, the effector function of T cells can be cytolytic activity or helper activity, including secretion of cytokines.

As used herein, the term "primary signaling domain" refers to a portion of protein that is capable of modulating primary activation of a TCR complex in a stimulatory manner or in an inhibitory manner. Primary signaling domains that act in a stimulatory manner typically contain signaling motifs known as immunoreceptor tyrosine-based activation motifs (ITAMs). Non-limiting examples of the ITAM containing primary signaling domain particularly useful in the present invention include those derived from TCRζ, FcRy, FcRβ, CD3y, CD3δ, CD3ε, CD3ζ, CD22, DAP10, CD79a, CD79b, and CD66d.

As used herein, the term "costimulatory signaling domain" refers to an intracellular signaling domain of a costimulatory molecule. A costimulatory molecule is a cell surface molecule, other than an antigen receptor or an Fc receptor, which provides a second signal required for highly efficient activation and function of T lymphocytes after binding to an antigen. Non-limiting examples of such costimulatory molecules include CARD11, CD2, CD7, CD27, CD28, CD30, CD40, CD54 (ICAM), CD83, CD134 (OX40), CD137 (4-1BB), CD150 (SLAMF1), CD270 (HVEM), CD278 (ICOS), DAP10.

As used herein, the term "immune effector cell" refers to any cell of the immune system that has one or more effector functions (e.g., cytotoxic cell-killing activity, secretion of cytokines, induction of ADCC and/or CDC). Typically, immune effector cells are cells of hematopoietic origin and that play a role in the immune response. The term "effector function" refers to a specialized function of an immune effector cell, such as a function or response that enhances or facilitates an immune attack on a target cell (e.g., killing a target cell, or inhibiting its growth or proliferation). The effector function of a T cell, for example, can be cytolytic activity or helper activity or activity including cytokine secretion. Examples of immune effector cells include T cells (e.g., α/β T cells and γ/δ T cells), B cells, natural killer (NK) cells, natural killer T (NKT) cells, mast cells, and bone marrow-derived macrophages.

The immune effector cell of the present invention can be self/autologous ("self") or non-self ("non-autologous", such as allogeneic, syngeneic or xenogeneic). As used herein, "self" refers to cells from the same subject; "allogeneic" refers to cells of the same species that are genetically different from the cell for comparison; "syngeneic" refers to cells from different subjects that genetically same with the cell for comparison; "xenogeneic" refers to cells from species different from the cell for comparison. In a preferred embodiment, the cells of the present invention are allogeneic.

Exemplary immune effector cell useful in the CAR described herein includes T lymphocyte. The term "T cell" or "T lymphocyte" is well known in the art and is intended to include thymocyte, immature T lymphocyte, mature T lymphocyte, resting T lymphocyte or activated T lymphocyte. The T cell may be T helper (Th) cell, such as T helper 1 (Th1) or T helper 2 (Th2) cell. The T cell can be helper T cell (HTL; CD4 T cell) CD4 T cell, cytotoxic T cell (CTL; CD8 T cell), CD4CD8 T cell, CD4CD8 T cell or any other subset of T cell. In certain embodiments, the T cell may include naive T cell and memory T cell.

Those of skilled in the art will appreciate that other cells can also be used as immune effector cells with the CAR as described herein, such as NK cells. Specifically, the immune effector cell also includes NK cell, monocyte, macrophage or dendritic cell, NKT cell, neutrophil, and macrophage. The immune effector cell also includes a progenitor cell of immune effector cell, wherein the progenitor cell can be induced in vivo or in vitro to differentiate into immune effector cells. Thus, in certain embodiments, the immune effector cell includes a progenitor cell of immune effector cell, such as hematopoietic stem cell (HSC) contained within a population of CD34+ cells derived from cord blood, bone marrow, or peripheral blood, which is differentiated into mature immune effector cells after administration, or it can be induced in vitro to differentiate into mature immune effector cells.

As used herein, the term "cytotoxic agent" comprises any agent that is detrimental to (e.g., kills) a cell, such as chemotherapeutic drug, bacterial toxin, plant toxin, or radioisotope, and the like.

As used herein, the term "vector" refers to a nucleic acid vehicle into which a polynucleotide can be inserted. When a vector enables the expression of a protein encoded by the inserted polynucleotide, the vector is referred to as an expression vector. A vector can be introduced into a host cell by transformation, transduction or transfection, so that the genetic material elements carried by the vector can be expressed in the host cell. Vectors are well known to those skilled in the art and include, but are not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage and animal viruses. Animal viruses that can be used as vectors include, but are not limited to, retroviruses (including lentiviruses), adenoviruses, adeno-associated viruses, herpes viruses (e.g., herpes simplex virus), poxviruses, baculoviruses, papillomaviruses, papovavirus (e.g., SV40). A vector may comprise a variety of elements that control expression, including, but not limited to, promoter sequence, transcription initiation sequence, enhancer sequence, selection element, and reporter gene. In addition, the vector may comprise a replication initiation site.

As used herein, in the term "episomal vector", "episomal" refers to that a vector can replicate without integrating into a chromosomal DNA of a host and will not be not gradually lost in dividing host cells, and also refers to that the vector is extrachromosomal or replicates independently.

As used herein, the term "viral vector" is used broadly to refer to a nucleic acid molecule (e.g., a transfer plasmid) that includes a virus-derived nucleic acid element that typically facilitates transfer or integration of the nucleic acid molecule into the genome of a cell, or a virus particle that mediates nucleic acid transfer. In addition to nucleic acids, viral particles will typically include various viral components, and sometimes host cell components. The term "viral vector" may refer to a virus or viral particle capable of transferring a nucleic acid into a cell, or to the transferred nucleic acid itself. Viral vectors and transfer plasmids contain structural and/or functional genetic elements derived primarily from viruses.

As used herein, the term "retroviral vector" refers to a viral vector or plasmid containing structural and functional genetic elements or fractions thereof derived primarily from a retrovirus.

As used herein, the term "lentiviral vector" refers to a viral vector or plasmid containing structural and functional genetic elements or fractions thereof (including LTR) derived primarily from a lentivirus. In certain embodiments, the terms "lentiviral vector", "lentiviral expression vector" may be used to refer to a lentiviral transfer plasmid and/or infectious lentiviral particle. Where elements (e.g., cloning site, promoter, regulatory element, heterologous nucleic acid, etc.) are referred to herein, it is to be understood that the sequences of these elements are present in the lentiviral particle of the present invention in RNA form, and in the DNA plasmid of the present invention in DNA form.

As used herein, an "integration-deficient" retrovirus or lentivirus refers to a retrovirus or lentivirus that has an integrase that is unable to integrate the viral genome into the genome of a host cell. In certain embodiments, the integrase protein is mutated to specifically reduce its integrase activity. Integration-deficient lentiviral vectors can be obtained by modifying the pol gene encoding an integrase protein to generate a mutant pol gene encoding an integration-deficient integrase. Such integration-deficient viral vectors have been described in patent application WO 2006/010834, which is incorporated herein by reference in its entirety.

As used herein, the term "host cell" refers to a cell that can be used to introduce a vector therein, which includes, but is not limited to, prokaryotic cell such as *E. coli* or *Bacillus subtilis,* fungal cell such as yeast cell or *Aspergillus,* etc., insect cell such as *S2 Drosophila* cell or Sf9, or animal cell such as fibroblast, CHO cell, COS cell, NSO cell, HeLa cell, BHK cell, HEK 293 cell or human cell, immune cell (e.g., T lymphocyte, NK cell, monocyte, macrophage or dendritic cell, etc.). The host cell may include a single cell or a population of cells.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the terms "conservative substitution" and "conservative amino acid substitution" refer to amino acid substitutions which would not disadvantageously affect or change the expected properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine) and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl Acad. Set USA 94: 412-417 (1997), which are incorporated herein by reference).

The 20 conventional amino acids involved herein are expressed in accordance with routine methods. See, for example, Immunology-A Synthesis (2nd Edition, E. S. Golub and D. R. Gren, Eds., Sinauer Associates, Sunderland, Mass. (1991)), which is incorporated herein by reference. In the inveniton, the terms "polypeptide" and "protein" have the same meanings, and can be used interchangeably. Moreover, in the invention, amino acids are generally expressed as one-letter codes and three-letter codes. For example, alanine may be expressed as A or Ala.

As used herein, the term "pharmaceutically acceptable carrier and/or excipient" refers to a carrier and/or excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995) and includes, but is not limited to: pH adjusting agents, surfactants, adjuvants, ionic strength enhancers, diluents, agents to maintain osmotic pressure, agents to delay absorption, preservatives. For example, pH adjusting agents include, but are not limited to, phosphate buffered saline. Surfactants include, but are not limited to, cationic, anionic or non-ionic surfactants, such as Tween-80. Ionic strength enhancers include, but are not limited to, sodium chloride. Preservatives include, but are not limited to, various antibacterial and antifungal agents, such as parabens, trichloro-t-butanol, phenol, sorbic acid, and the like. Agents to maintain osmotic pressure include, but are not limited to, sugar, NaCl, and the like. Agents to delay absorption include, but are not limited to, monostearate and gelatin. Diluents include, but are not limited to, water, aqueous buffers (e.g., buffered saline), alcohols and polyols (e.g., glycerol), and the like. Preservatives include, but are not limited to, various antibacterial and antifungal agents, such as thimerosal, 2-phenoxyethanol, parabens, trichloro-t-butanol, phenol, sorbic acid, and the like. Stabilizers have the meaning commonly understood by those skilled in the art, which can stabilize the desired activity of active ingredient in drug, including but not limited to sodium glutamate, gelatin, SPGA, saccharides (e.g., sorbitol, mannitol, starch, sucrose, lactose, dextran, or glucose), amino acids (e.g., glutamic acid, glycine), proteins (e.g., dried whey, albumin, or casein) or degradation products thereof (e.g., lactalbumin hydrolysate), etc. In certain exemplary embodiments, the pharmaceutically acceptable carrier or excipient includes a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

As used herein, the term "prevention/preventing" refers to a method performed to prevent or delay the occurrence of a disease or disorder or symptom (e.g., a tumor) in a subject. As used herein, the term "treatment/treating" refers to a method performed to obtain a beneficial or desired clinical result. For the purposes of the present invention, the beneficial or desired clinical result includes, but is not limited to, easing symptom, reducing the scope of disease, stabilizing (i.e., no longer exacerbating) the state of disease, delaying or slowing the development of disease, improving or alleviating the status of disease, and alleviating a symptom (either in part or in whole), either detectable or undetectable. In addition, the term "treatment/treating" can also refer to prolonging survival period compared to the expected survival period (if not receiving treatment).

As used herein, the term "subject" refers to a mammal, such as a primate, such as a human. In certain embodiments, the term "subject" is meant to include a living organism in which an immune response can be elicited. In certain embodiments, the subject (e.g., human) has, or is at risk for, a B-cell related disease (e.g., B-cell malignancy).

As used herein, the term "effective amount" refers to an amount sufficient to obtain, or at least partially obtain, the desired effect. For example, a prophylactically effective amount for a disease (e.g., B cell-related disease) refers to an amount sufficient to prevent, stop, or delay the onset of a disease (e.g., B cell-related disease); a therapeutically effective amount for a disease refers to an amount sufficient to cure or at least partially prevent the disease and its complications in a patient who already has the disease. Determining such effective amounts is well within the ability of those skilled in the art. For example, a therapeutically effective amount will depend on the severity of the disease to be treated, the general state of the patient's own immune system, the patient's general condition such as age, weight and sex, the mode of administration of the drug, and other concurrently administered therapies, etc.

As used herein, the term "B cell-related disease" refers to a disease condition involving inappropriate B cell activity and B cell malignancy, including but not limited to B cell malignancy or B cell related autoimmune disease. As used herein, the term "B cell malignancy" includes a cancer type that develops in B cells (a type of immune system cell), for example, multiple myeloma (MM) and non-Hodgkin's lymphoma (NHL).

### Beneficial effects of the present invention

The present invention provides a BCMA-targeting humanized antibody, which can significantly reduce the non-specific binding to normal tissue cells while maintaining the activity of binding to BCMA-expressing tumor cells equivalent to that of the mouse antibody, which is beneficial to reducing non-specific toxicity, and improving drug safety. The present invention further provides a BCMA-targeting chimeric antigen receptor comprising the humanized antibody. The immune effector cells expressing the chimeric antigen receptor of the present invention basically do not cause antigen-independent cytokine release, and do not induce continuous activation and exhaustion of T cells, and have significantly improved safety. Therefore, the humanized antibody and CAR of the present invention have the potential for preventing and/or treating a B cell-related disease (e.g., B cell malignancy, autoimmune disease, etc.), and have great clinical values.

The embodiments of the present invention will be described in detail below with reference to the drawings and examples, but those skilled in the art will understand that the following drawings and examples are only used to illustrate the present invention, rather than limit the scope of the present invention. The various objects and advantageous aspects of the present invention will become apparent to those skilled in the art from the accompanying drawings and the following detailed description of the preferred embodiments.

### Brief Description of the Drawings

FIG. 1 shows the binding curves of humanized antibodies C11D5.3-03, C11D5.3-04 and parental murine antibody C11D5.3 to recombinant human BCMA protein.
FIG. 2 shows the dose-dependent binding curves of humanized antibodies C11D5.3-03, C11D5.3-04 and parental murine antibody C11D5.3 to human multiple myeloma cell line U266.
FIG. 3 shows the dose-dependent binding curves of humanized antibodies C11D5.3-03, C11D5.3-04 and parental murine antibody C11D5.3 to human multiple myeloma cell line MM.IS.
FIG. 4 shows the dose-dependent binding curves of humanized antibodies C11D5.3-03, C11D5.3-04 and parental murine antibody C11D5.3 to human multiple myeloma cell line RPMI8226.
FIG. 5 shows the dose-dependent binding curves of humanized antibodies C11D5.3-03, C11D5.3-04 and parental murine antibody C11D5.3 to human Burkitt's lymphoma cell Daudi that does not express BCMA.
FIG. 6 shows the binding curves of humanized antibodies C11D5.3-03, C11D5.3-04 and parental murine antibody C11D5.3 to human immortalized keratinocyte cell line Hacat.
FIG. 7 shows the binding curves of humanized antibodies C11D5.3-03, C11D5.3-04 and parental murine antibody C11D5.3 to human gastric mucosa epithelial cell line GES.
FIG. 8 shows the expression of BCMA-CAR on T cells transduced with C11D5.3-CAR, or humanized C11D5.3-03-CAR and C11D5.3-04-CAR.
FIG. 9 shows the IFNγ release assay results of C11D5.3 CAR-T or humanized C11D5.3-03 CAR-T, C11D5.3-04 CAR-T.
FIG. 10 shows the IL2 release assay results of C11D5.3 CAR-T or humanized C11D5.3-03 CAR-T, C11D5.3-04 CAR-T.
FIG. 11 shows the LDH release assay results of U266 killing caused by C11D5.3 CAR-T or humanized C11D5.3-03 CAR-T and C11D5.3-04 CAR-T.
FIG. 12 shows the LDH release assay results of Daudi killing caused by C11D5.3 CAR-T or humanized C11D5.3-03 CAR-T and C11D5.3-04 CAR-T.
FIGS. 13-17 show respectively the detection results of the release levels of IL4 (FIG. 13), IL6 (FIG. 14), IL10 (FIG. 15), TNFα (FIG. 16) and IFNγ (FIG. 17) of C11D5.3 CAR-T or humanized C11D5.3-03 CAR-T, C11D5.3-04 CAR-T and untransfected PBMC cells in the serum-free culture system in the absence of antigen stimulation.
FIG. 18 shows the detection results of the release levels of IL4, IL6, IL10, TNFα and IFNγ of C11D5.3 CAR-T or humanized C11D5.3-03 CAR-T, C11D5.3-04 CAR-T and untransfected PBMC cells in the culture system containing 5% human serum in the absence of antigen stimulation.
FIG. 19 shows the detection results of the expression level of HLA-DR on the surface of CAR-T cells.
FIG. 20 shows the detection results of the expression level of CD25 on the surface of CAR-T cells.
FIG. 21 shows the detection results of the expression levels of activated Caspase-3 in C11D5.3-03 CAR-T, C11D5.3-04 CAR-T, C11D5.3 CAR-T and the control PBMC.

### Sequence Information

Information on the partial sequences involved in the present invention is provided in Table 1 below.

**Table 1: Description of sequences**

| SEQ ID NO | Description | SEQ ID NO | Description |
|---|---|---|---|
| 1 | C11D5.3-03 VH | 9 | CD3ζ ITAM |
| 2 | C11D5.3-03 VL | 10 | C11D5.3-03-CAR full-length amino acid sequence |
| 3 | C11D5.3-04 VH | 11 | nucleic acid sequence encoding C11D5.3-03-CAR |
| 4 | C11D5.3-04 VL | 12 | C11D5.3-04-CAR full-length amino acid sequence |
| 5 | Linker | 13 | Nucleic acid sequence encoding C11D5.3-04-CAR |
| 6 | Hinge region | 14 | C11D5.3-CAR full-length amino acid sequence |
| 7 | Transmembrane region | 15 | Nucleic acid sequence encoding C11D5.3-CAR |
| 8 | CD137 intracellular signaling domain | 16 | Nucleic acid sequence encoding P2A-tEGFR |

### EXAMPLES

The present invention will now be described with reference to the following examples, which are intended to illustrate, but not limit, the present invention.

Unless otherwise specified, the molecular biology experimental methods and immunoassays used in the present invention basically refer to J. Sambrook et al., Molecular Cloning: A Laboratory Manual, 2nd Edition, Cold Spring Harbor Laboratory Press, 1989, and FM Ausubel et al., Refined Laboratory Guide for Molecular Biology, 3rd Edition, John Wiley & Sons, Inc., 1995; restriction enzymes were used according to the conditions recommended by the product manufacturer. Those skilled in the art appreciate that the examples describe the present invention by way of example and are not intended to limit the scope of the present invention as claimed.

### Example 1: Humanization of anti-BCMA murine antibody and preparation of humanized antibody

In order to improve the sequence homology between the antibody and the human antibody and to reduce the immunogenicity of the antibody to human, the mouse antibody C11D5.3 (of which the heavy chain variable region sequence and light chain variable region sequence referred to SEQ ID NO: 3 and SEQ ID NO: 12 in CN 201510142069.2, respectively) were subjected to the design for humanization by using methods known in the art to insert murine CDR regions into human framework sequences (see US Patent No. 5,225,539 to Winter; U.S. Patent Nos. 5,530,101; 5,585,089; 5,693,762 and 6,180,370 to Queen et al.; and Lo, Benny, KC, editor, in Antibody Engineering: Methods and Protocols, volume 248, Humana Press, New Jersey, 2004). Generally, all or part of the CDR regions of a humanized antibody are derived from a non-human antibody (donor antibody), and all or part of the non-CDR regions (e.g., variable region FRs and/or constant regions) are derived from human immunoglobulin (receptor antibody). According to the above method and after extensive screening, two humanized antibodies (scFv) were constructed based on the CDRs of the murine antibody C11D5.3, named C11D5.3-03 and C11D5.3-04 respectively. The VH and VL of C11D5.3-03 were set forth in SEQ ID NO: 1 and SEQ ID NO: 2, respectively; the VH and VL of C11D5.3-04 were set forth in SEQ ID NO: 3 and SEQ ID NO: 4, respectively. In addition, the murine antibody C11D5.3 (scFv) was prepared as a reference antibody.

The HEK293-F cells (Invitrogen, R7907) transiently transfected with expression plasmids encoding the above antibodies were cultured in Freestyle 293 expression medium (Invitrogen, 12338018) and incubated in a CO₂ incubator at 37°C for 5-7 days. Cells and cell debris were removed by centrifugation, and the culture supernatant was collected, filtered through a 0.22 µm membrane. Purification was performed by using a protein A column (GE Life Science, 17127901) according to the manufacturer's instructions, in which after washing with PBS, an acid buffer (pH 3.5) containing 20 mM citrate and 150 mM NaCl was used to elute antibodies, and the eluate was neutralized with 1M Tris (pH 8.0), and dialyzed against PBS. Antibody concentration was determined by measuring absorbance at 280 nm, and aliquoted antibodies were stored in a -80°C refrigerator.

### Example 2: Affinity assessment of anti-BCMA humanized antibodies to recombinant human BCMA protein

The binding activity of the antibodies to human BCMA protein was assessed using an ELISA method, and the antibody potency was compared using dose-dependent binding curves in ELISA.

BCMA protein (Sino Biological, 10620-H08H) was diluted to 1 µg/mL in PBS, 100 µL of the protein solution was added to each well of the ELISA plate and incubated at 4°C overnight. The plate was washed 3 times in wash buffer (PBS, 0.05% Tween 20), and non-specific sites were blocked by adding 200 µl/w PBS + 2% BSA blocking solution. C11D5.3-03, C11D5.3-04 or murine antibody C11D5.3 was added and incubated at 37°C for 2h. The plate was washed 3 times in wash buffer, and HRP-conjugated goat anti-human secondary antibody was added and incubated for 1 h at room temperature. The plate was washed 3 times in wash buffer, and the bound secondary antibody was detected by adding TMB (HRP substrate) and incubating the plate in the dark at room temperature for 5 to 10 minutes. The enzymatic reaction was stopped by adding 1M of sulfuric acid solution, and the light absorption at 450 nm was measured.

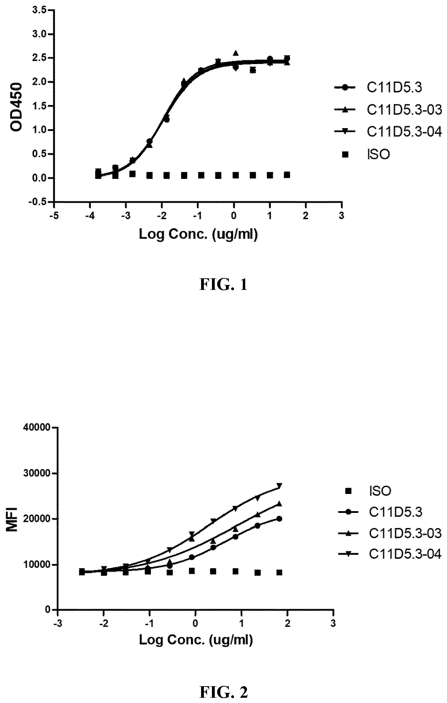

The results were shown in FIG. 1. The humanized antibodies C11D5.3-03 and C11D5.3-04 had EC50 values (effective concentration at 50% activity) of 0.011 µg/mL and 0.011 µg/mL, respectively, indicating they had equivalent binding activity compared with the murine antibody C11D5.3 (EC50=0.011 µg/mL).

### Example 3: Affinity assessment of anti-BCMA humanized antibodies to BCMA-expressing tumor cells

The binding activity of the humanized antibodies obtained in Example 1 to BCMA-expressing cells was evaluated using the FACS method, and the antibody potency was compared using the dose-dependent binding curves in FACS.

Cells (human myeloma cells expressing BCMA or tissue cells not expressing BCMA) were digested, and 500,000 cells were seeded in 50 µL of PBS containing 2% FBS (FACS buffer) in a U-bottom 96-well plate. The test samples were subjected to a 3-fold serial dilution by diluting 1/3 volume (100 µL) in 200 µL of FACS buffer, starting at a concentration of 300 µg/mL (final concentration). 50 µL of the diluted antibody was added to each well of the cell plate and incubated at 4°C for 1 hour. After washing twice with FACS buffer, 100 µL of secondary antibody (Abcam ab97003, 5 µg/mL, diluted in FACS buffer) was added to each well, and incubated at 4°C for 0.5 hours. After staining and washing twice with FACS buffer, 200 µL of FACS buffer was added to each well, and reading was performed on the machine (BD C6plus).

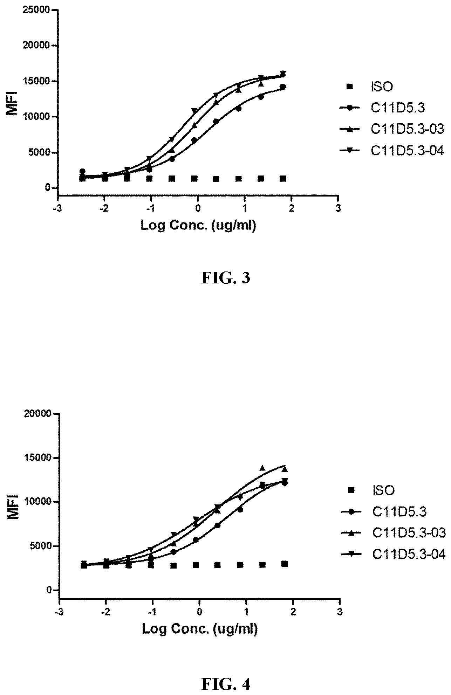

FIGS. 2, 3, 4 and 5 showed the dose-dependent binding curves of the antibodies to human multiple myeloma cells U266, MM.1S, RPMI8226, and human Burkitt's lymphoma cell Daudi that did not express BCMA, respectively. The EC50 values were shown in Table 2. The results showed that for the three BCMA-expressing tumor cells, the humanized antibodies C11D5.3-03 and C11D5.3-04 had a high affinity comparable to that of the murine antibody; in the meantime, for the tumor cells not expressing BCMA, the humanized antibodies C11D5.3-03 and C11D5.3-04 also exhibited a low affinity comparable to that of the murine antibody.

**Table 2: EC50 values of anti-BCMA antibodies binding to BCMA-expressing tumor cells**

| | EC50 (µg/ml) | | |
|---|---|---|---|
| | C11D5.3 | C11D5.3-03 | C11D5.3-04 |
| U266 | 4.4 | 7.57 | 1.99 |
| MM.1S | 1.62 | 0.81 | 0.47 |
| RPMI8226 | 3.81 | 2.25 | 0.82 |
| Daudi | 58.25 | 36.32 | 30.71 |

### Example 4: Evaluation of non-specific binding of anti-BCMA humanized antibodies to non-tumor tissue cells

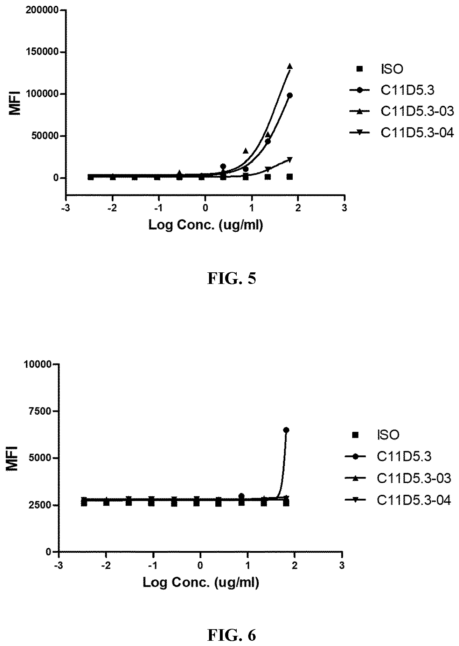

The non-specific binding between the purified humanized antibodies obtained in Example 1 and non-tumor tissue cells was evaluated using FACS method, and using tissue cells that did not express BCMA, and the specific method was as described above. FIGS. 6-7 showed the binding curves of the antibodies to human immortalized keratinocyte Hacat and human gastric mucosal epithelial cell GES, respectively. As shown in the figures, the murine antibody C11D5.3 bound to both Hacat and GES cells at high concentration (100 µg/mL), in contrast, the two humanized antibodies C11D5.3-03 and C11D5.3-04 did not bind to either of the two non-tumor tissue cells at the concentrations tested (0.003 to 100 µg/mL). The above results showed that the humanized antibodies of the present invention had reduced non-specific binding compared to the murine antibody C11D5.3, and such a technical effect was significant and unexpected.

### Example 5: Construction and preparation of chimeric antigen receptor (CAR) lentiviral expression vector

Based on the humanized antibodies obtained in the above example, CAR lentiviral expression vectors were further constructed. In the constructed chimeric antigen receptor lentiviral expression vectors, the amino acid sequences of elements from the N-terminus to the C-terminus were shown in Table 3 below. The CAR comprised the scFv (VH+VL) of the humanized antibody described above, CD8α hinge region, CD8α transmembrane region, CD137 intracellular domain, and CD3ζ ITAM region. The nucleic acid sequence encoding the above CAR was linked with the sequence (SEQ ID NO: 16) encoding P2A-tEGFR and inserted into the GV401 vector (Genechem), with the frame of EFla Promoter-BCMA CAR-P2A-tEGFR-WPRE, and the BCMA CAR expression was monitored by detecting tEGFR expression.

**Table 3: Amino acid sequences of elements from N-terminal to C-terminal in chimeric antigen receptors**

| CAR name | N-terminal → C-terminal, amino acid sequence of each element (SEQ ID NO: ) | | | | | | | Full-length sequence | Encoding nucleic acid sequence |
|---|---|---|---|---|---|---|---|---|---|
| | VH | Linker | VL | Hinge region | Transmemb rane region | CD137 | CD3ζ | | |
| C11D5.3-03 | 1 | 5 | 2 | 6 | 7 | 8 | 9 | 10 | 11 |
| C11D5.3-04 | 3 | | 4 | | | | | 12 | 13 |
| C11D5.3 | SEQ ID NO:3 of CN20151014206 9.2 | | SEQ ID NO:12 of CN20151014206 9.2 | | | | | 14 | 15 |

### Example 6: Preparation of CAR-T cells

PBMC cells were collected from healthy people (Xuanfeng Bio, SLB-HP010); 1 µg/ml CD3 antibody (Miltenyi, 170-076-124) and 1 µg/ml CD28 antibody (Miltenyi, 170-076-117) were coated in a 6-well plate; PBMC was added to the coated 6-well plate and incubated overnight. The CAR lentiviral vectors described in Example 5 were added to PBMC at MOI=3, and centrifuged at 2000rpm for 60 minutes for transduction, thereby obtaining the T cells transduced with murine C11D5.3-CAR (C11D5.3 CAR-T), the T cells transduced with humanized C11D5.3-03-CAR (C11D5.3-03 CAR-T), and the T cells transduced with humanized C11D5.3-04-CAR (C11D5.3-04 CAR-T). After 48 hours of culture, tEGFR expression was detected with Cetuximab labeled with PE fluorescence.

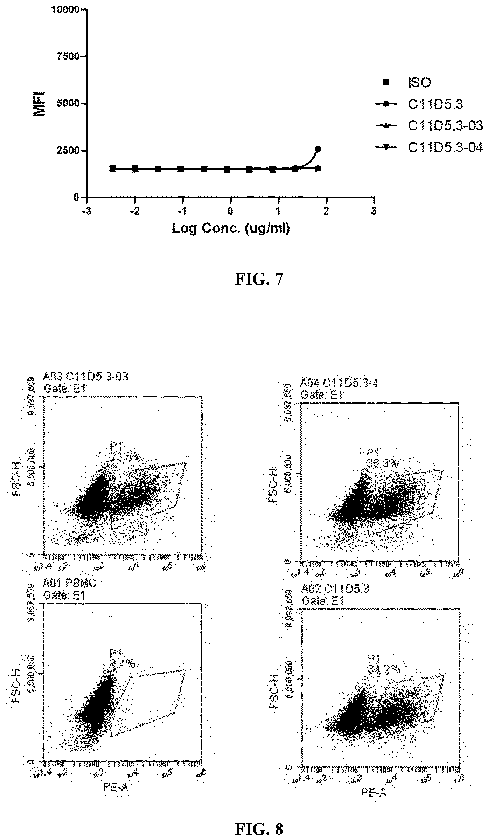

FIG. 8 showed the expression of BCMA-CAR on untransfected control PBMC and the above three CAR-T cells. The results showed that all the above three CAR-Ts could express the corresponding anti-BCMA CAR, indicating that the anti-BCMA CAR-T was successfully constructed.

### Example 7: Evaluation of in vitro anti-tumor ability of anti-BCMA CAR-T

Cytokine release assay: The anti-BCMA CAR-T cells prepared in Example 6 were collected, washed twice with DPBS solution, and resuspended in RPMI1640 medium containing 2% FBS; the tumor cells were collected, and then washed and resuspended in RPMI1640 medium containing 2% FBS, the tumor cells included: tumor cells that did not express BCMA (293T, K562), tumor cells that expressed BCMA (M1ss, U266, RPMI8226, Daudi).

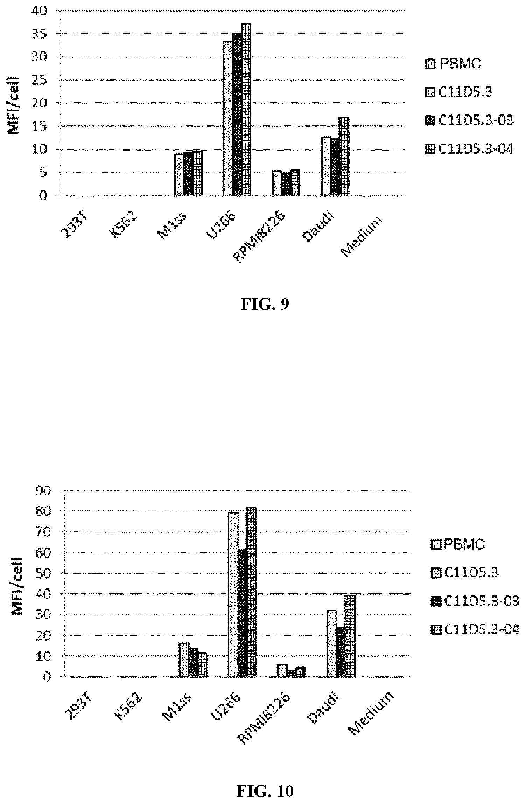

The anti-BCMA CAR-T cells and the above tumor cell lines were mixed and cultured at E:T (effector cells/target cells) = 1:1 for 16 hours, and supernatants were collected and the release of cytokines was determined by BD CBA assay. The IFNγ and IL2 release results of the control PBMC, C11D5.3 CAR-T, C11D5.3-03 CAR-T, and C11D5.3-04 CAR-T were shown in FIG. 9 and FIG. 10, respectively.

The above results showed that the humanized C11D5.3-03 CAR-T and C11D5.3-04 CAR-T were similar to the murine C11D5.3 CAR-T, which could specifically recognize the BCMA+ cell line and release IFNγ and IL2.

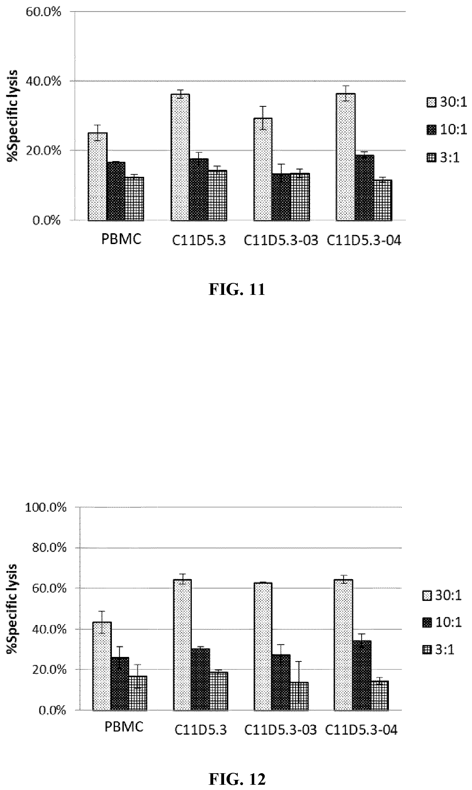

Tumor cell lysis experiment: BCMA-expressing cell lines (U266, Daudi) and anti-BCMA CAR-T cells were mixed and cultured for 4 hours according to E:T=30:1, 10:1, and 3:1, respectively, and the supernatants were collected to detect LDH release (CytoTox96 NonRadioactive Cytotoxicity Assay). The LDH release results of U266 and Daudi killed by the control PBMC, C11D5.3 CAR-T, C11D5.3-03 CAR-T, C11D5.3-04 CAR-T were shown in FIG. 11 and FIG. 12, respectively.

The above results showed that the humanized C11D5.3-03 CAR-T and C11D5.3-04 CAR-T were similar to the murine C11D5.3 CAR-T, and could recognize and kill BCMA+ tumor cells.

### Example 8: Cytokine release of humanized anti-BCMA CAR-T cells

It was reported that anti-BCMA CAR-T cells could release cytokines in the absence of BCMA antigen, and this release level varied with the structural differences in anti-BCMA CAR molecules; the toxicity caused by enhanced cytokine release was one of the known toxicity problems of anti-BCMA CAR T cell clinical therapy. It was known that CAR-T cell therapy based on murine antibody C11D5.3 had not found significant enhanced cytokine release problems in the current clinical practice. However, the study further found that some humanized anti-BCMA CARs caused antigen-independent cytokine release, which would make humanized anti-BCMA CARs unsuitable for T-cell therapy. To this end, this example evaluated the antigen-independent cytokine release of humanized anti-BCMA CAR-T cells.

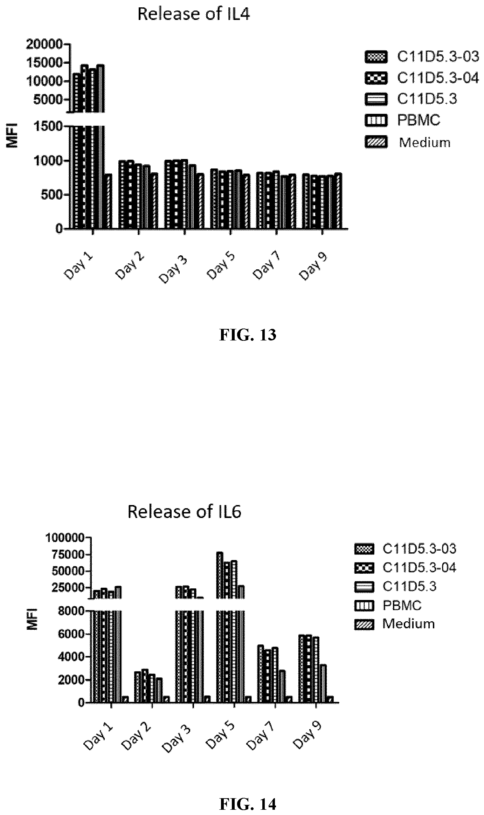

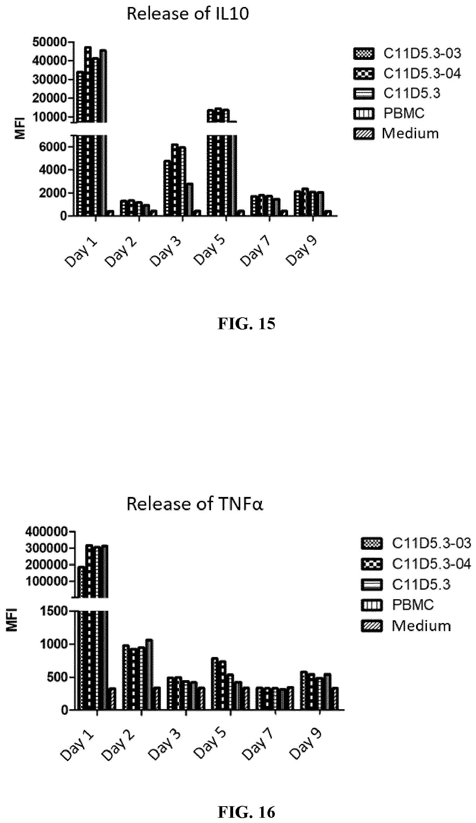

The CAR-T obtained in Example 6 or PBMC control was statically cultured in AIM-V culture medium (Gibco, A3021002) containing 60 IU/ml IL2 (Miltenyi, 130-097-748) in an incubator at 5% CO₂ and 37°C, and the cytokine release in the absence of stimulation was determined at various incubation time points. The control PBMC, C11D5.3 CAR-T, C11D5.3-03 CAR-T, or C11D5.3-04 CAR-T was cultured, supernatant was collected on the first day (before the first medium change), the second day, the third day, the fifth day (before the second medium change), the seventh day, and the ninth day, and the multiplexed magnetic bead method (TH1/TH2 cytokine CBA kit, BD, #550749) was used to determine the release levels of 5 cytokines released into the supernatant, including IL4, IL6, IL10, TNFα and IFNγ. FIGS. 13-17 showed the comparison of the release levels of IL4, IL6, IL10, TNFα and IFNγ at different culture time points for the above three CAR-T cells and untransfected PBMC cells in the serum-free culture system without antigen stimulation, respectively. It could be seen that in the absence of stimulation, the release of type 1 inflammatory factors of humanized C11D5.3-03 CAR-T and C11D5.3-04 CAR-T did not change significantly compared with the untransfected PBMC cells.

The above results indicated that the humanized anti-BCMA CAR-T cells of the present invention did not induce significant antigen-independent cytokine release.

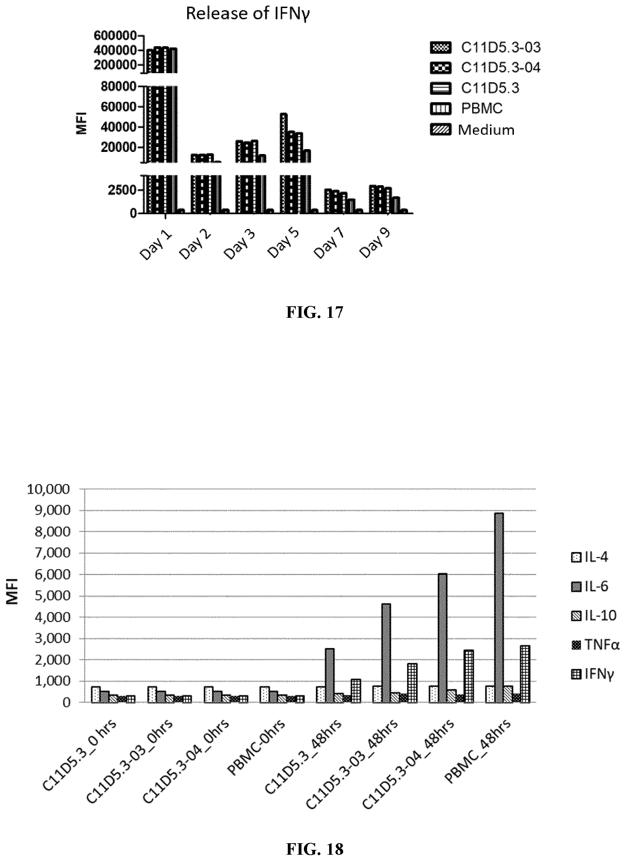

In AIM-V medium (Gibco, A3021002) containing human serum (Corning, MT35060CI) but without BCMA antigen, 1×10⁵ cells of control PBMC, C11D5.3 CAR-T, C11D5.3-03 CAR-T, or C11D5.3-04 CAR-T were cultured for 48 hours, and the multiplexed magnetic bead method was used to determine the levels of 5 cytokines released into the supernatant, including IL4, IL6, IL10, TNFα and IFNγ. The release cytokines of the above three CAR-T cells were compared to assess whether sequence changes in C11D5.3 caused by humanization would introduce a new reactivity to proteins in human serum that was not seen in mouse anti-BCMA CAR-T cells. FIG. 18 showed a comparison of release levels of IL4, IL6, IL10, TNFα and IFNγ for the above three CAR-T cells and the untransfected PBMC cells in the absence of antigen stimulation in a culture system containing 5% human serum. It could be seen that the release of type 1 inflammatory factors of the two humanized anti-BCMA CAR-T cells did not change significantly compared with the murine CAR-T cells, and did not change significantly in terms of the release of type 1 inflammatory factors compared with the untransfected PBMC cells either.

The above results indicated that the sequence changes to C11D5.3 caused by the humanization of C11D5.3-03 and C11D5.3-04 did not introduce a new reactivity to proteins in human serum that was not seen in mouse anti-BCMA CAR-T cells.

In sum, the CARs obtained based on the humanized antibodies C11D5.3-03 and C11D5.3-04 of the present invention could reduce the potential immunogenicity caused by the immune response of the mouse sequence, while showing no obvious antigen-independent cytokine release and new reactivity to proteins in human serum, thereby having significantly improved safety. Such technical effects were remarkable and unexpected.

### Example 9: Evaluation of activation status of humanized anti-BCMA CAR T cells

The CAR-T or PBMC control prepared in Example 6 were cultured in the culture system described in Example 8, the expression of T cell activation phenotype markers on the surface of the three CAR-T cells were detected on the fifth day and the ninth day (end point) of culturing, to assess whether sequence changes to C11D5.3 caused by humanization would introduce hyperactivation of CAR-T cells that was not seen in mouse anti-BCMA CAR-T cells. The expression levels of HLA-DR (APC anti-human HLA-DR Antibody, Biolegend) and CD25 (FITC anti-human CD25 Antibody, Biolegend) on the surface of CAR T cells were detected by FACS method.

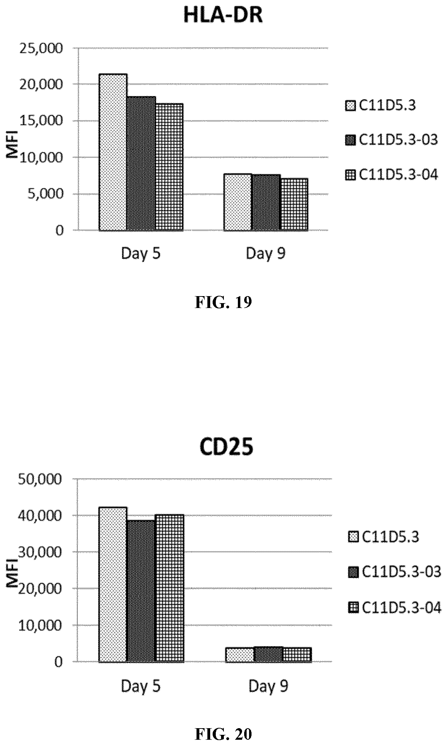

The results were shown in FIG. 19 and FIG. 20, respectively. The results showed that the humanized C11D5.3-03 CAR-T and C11D5.3-04 CAR-T did not show hyperactivation of CAR-T cells compared with the murine C11D5.3 CAR-T.

### Example 10: Apoptosis evaluation of humanized anti-BCMA CAR T cells

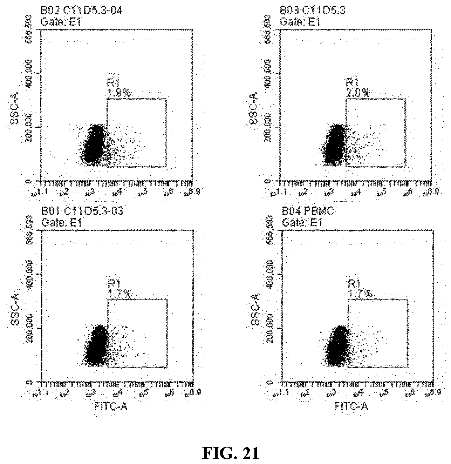

The exhaustion of T cells is often associated with the activation-induced cell death caused by apoptosis. Levels of activated Caspase-3 (CaspGLOW^{™} Fluorescein Active Caspase-3 Staining Kit, Invitrogen) were measured to examine whether the introduction of anti-BCMA CAR could lead to higher levels of T cell apoptosis. The expression levels of activated Caspase-3 in C11D5.3-03 CAR-T, C11D5.3-04 CAR-T, C11D5.3 CAR-T and the control PBMC were detected by FACS method, and the results were shown in FIG. 21. The results showed that the introduction of humanized C11D5.3-03 CAR and C11D5.3-04 CAR did not affect the expression level of activated Caspase-3 in T cells, and did not lead to cell death caused by T cell apoptosis.

Although specific embodiments of the present invention have been described in detail, those skilled in the art will appreciate that various modifications and changes can be made to the details in light of all the teachings that have been published, and that these changes are all within the scope of the present invention. The full scope of the present invention is given by the appended claims and any equivalents thereof.

### References:

1. Pieper K, Grimbacher B, Eibel H. B-cell biology and development. J Allergy Clin Immunol. 2013; 131(4):959-71
2. Kalled SL, Ambrose C, Hsu YM. The biochemistry and biology of BAFF, APRIL and their receptors. Curr Dir Autoimmun. 2005; 8:206-242
3. Zhang X, Park CS, Yoon SO, Li L, Hsu YM, Ambrose C, Choi YS. BAFF supports human B cell differentiation in the lymphoid follicles through distinct receptors. Int Immunol. 2005;17:779-788
4. Carpenter RO, Evbuomwan MO, Pittaluga S, Rose JJ, Raffeld M, Yang S, Gress RE, Hakim FT, Kochenderfer JN. B-cell maturation antigen is a promising target for adoptive T-cell therapy of multiple myeloma. Clin Cancer Res.2013;19(8):2048-60
5. Xu S, Lam KP. B-cell maturation protein, which binds the tumor necrosis factor family members BAFF and APRIL, is dispensable for humoral immune responses. Mol Cell Biol. 2001; 21:4067-4074
6. Brian PO, Vanitha SR, Loren DE, George TM, Richard JB, Randolph JN. BCMA Is Essential for the Survival of Long-lived Bone Marrow Plasma Cells. J Exp Med. 2004; 199(1):91-8
7. Jerome M, Eric L, Eric Jourdan, Philippe Q, Thierry R, Cecile L, Philippe M, Bernard K, Karin T. BAFF and APRIL protect myeloma cells from apoptosis induced by interleukin 6 deprivation and dexamethasone. Blood. 2004; 103 (8): 3148-3157
8. Palumbo A, Anderson K. Multiple myeloma. N Engl J Med. 2011; 364(11): 1046-60
9. Siegel R, Ma J, Zou Z, Jemal A. Cancer statistics, 2014. CA- Cancer J Clin. 2014; 64(1):.9-29

## Claims

1. An antibody or antigen-binding fragment thereof capable of specifically binding to BCMA, the antibody or antigen-binding fragment thereof comprising:
(a) a heavy chain variable region (VH) comprising an amino acid sequence selected from the group consisting of:
(i) a sequence set forth in SEQ ID NO: 1 or 3;
(ii) a sequence having a substitution, deletion or addition of one or several amino acids (e.g., substitution, deletion or addition of 1, 2, 3, 4 or 5 amino acids) compared to the sequence set forth in SEQ ID NO: 1 or 3; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 1 or 3;
and/or,
(b) a light chain variable region (VL) comprising an amino acid sequence selected from the group consisting of:
(iv) a sequence set forth in SEQ ID NO: 2 or 4;
(v) a sequence having a substitution, deletion or addition of one or several amino acids (e.g., substitution, deletion or addition of 1, 2, 3, 4 or 5 amino acids) compared to the sequence set forth in SEQ ID NO: 2 or 4; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 2 or 4;
preferably, the substitution described in (ii) or (v) is a conservative substitution.

2. The antibody or antigen-binding fragment thereof according to claim 1, wherein the antibody or antigen-binding fragment thereof comprises:
(a) a heavy chain variable region (VH) comprising an amino acid sequence selected from the group consisting of:
(i) a sequence set forth in SEQ ID NO: 1;
(ii) a sequence having a substitution, deletion or addition of one or several amino acids (e.g., substitution, deletion or addition of 1, 2, 3, 4 or 5 amino acids) compared to the sequence set forth in SEQ ID NO: 1; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 1;
and,
(b) a light chain variable region (VL) comprising an amino acid sequence selected from the group consisting of:
(iv) a sequence set forth in SEQ ID NO: 2;
(v) a sequence having a substitution, deletion or addition of one or several amino acids (e.g., substitution, deletion or addition of 1, 2, 3, 4 or 5 amino acids) compared to the sequence set forth in SEQ ID NO: 2; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 2;
preferably, the substitution described in (ii) or (v) is a conservative substitution;
preferably, the antibody or antigen-binding fragment thereof comprises a heavy chain variable region (VH) and a light chain variable region (VL), the VH comprising a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 2 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto.

3. The antibody or antigen-binding fragment thereof according to claim 1, wherein the antibody or antigen-binding fragment thereof comprises:
(a) a heavy chain variable region (VH) comprising an amino acid sequence selected from the group consisting of:
(i) a sequence set forth in SEQ ID NO: 3;
(ii) a sequence having a substitution, deletion or addition of one or several amino acids (e.g., substitution, deletion or addition of 1, 2, 3, 4 or 5 amino acids) compared to the sequence set forth in SEQ ID NO: 3; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 3;
and,
(b) a light chain variable region (VL) comprising an amino acid sequence selected from the group consisting of:
(iv) a sequence set forth in SEQ ID NO: 4;
(v) a sequence having a substitution, deletion or addition of one or several amino acids (e.g., substitution, deletion or addition of 1, 2, 3, 4 or 5 amino acids) compared to the sequence set forth in SEQ ID NO: 4; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence set forth in SEQ ID NO: 4;
preferably, the substitution described in (ii) or (v) is a conservative substitution;
preferably, the antibody or antigen-binding fragment thereof comprises a heavy chain variable region (VH) and a light chain variable region (VL), the VH comprising a sequence set forth in SEQ ID NO: 3 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 4 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto.

4. The antibody or antigen-binding fragment thereof according to any one of claims 1 to 3, wherein the antibody or antigen-binding fragment thereof further comprises a heavy chain constant region (CH) and a light chain constant region (CL);
preferably, the heavy chain constant region is selected from the group consisting of IgG, IgM, IgE, IgD or IgA;
preferably, the heavy chain constant region is an IgG heavy chain constant region, such as an IgGl, IgG2, IgG3 or IgG4 heavy chain constant region;
preferably, the light chain constant region is selected from κ or λ;
preferably, the light chain constant region is a κ light chain constant region.

5. The antibody or antigen-binding fragment thereof according to any one of claims 1 to 4, wherein the antibody or antigen-binding fragment thereof is selected from the group consisting of scFv, di-scFv, (scFv)₂, Fab, Fab', (Fab')₂, Fv, disulfide-linked Fv.

6. A chimeric antigen receptor (CAR), which comprises an extracellular antigen-binding domain, a spacer domain, a transmembrane domain and an intracellular signaling domain, wherein the extracellular antigen-binding domain comprises the antibody or antigen-binding fragments thereof according to any one of claims 1 to 5;
preferably, the antibody or antigen-binding fragment thereof is selected from the group consisting of Fab fragment, Fab' fragment, F(ab)'₂ fragment, Fv, disulfide-stabilized Fv protein ("dsFv"), scFv, di-scFv, (scFv)₂;
preferably, the antibody or antigen-binding fragment thereof is selected from the group consisting of scFv, di-scFv, (scFv)₂.

7. The chimeric antigen receptor according to claim 6, wherein the extracellular antigen-binding domain comprises a heavy chain variable region (VH) and a light chain variable region (VL), the VH comprising a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 2 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto;
preferably, the VH and VL are connected by a linker; preferably, the linker has a sequence set forth in SEQ ID NO:5.

8. The chimeric antigen receptor according to claim 6, wherein the extracellular antigen-binding domain comprises a heavy chain variable region (VH) and a light chain variable region (VL), the VH comprising a sequence set forth in SEQ ID NO: 3 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% compared thereto, the VL comprising a sequence set forth in SEQ ID NO: 4 or a sequence having a sequence identity of at least 80%, at least 85%, at least 90%, at least 91%, at least 92%, at least 93% compared to the %, at least 94%, at least 95%, at least 96%, at least 97%, at least 98%, at least 99%, or 100% compared thereto;
preferably, the VH and VL are connected by a linker; preferably, the linker has a sequence set forth in SEQ ID NO:5.

9. The chimeric antigen receptor according to any one of claims 6 to 8, wherein the spacer domain is selected from the group consisting of a hinge domain and/or CH2 and CH3 regions of an immunoglobulin (e.g., IgG1 or IgG4);
preferably, the spacer domain comprises a hinge region of CD8α;
preferably, the spacer domain comprises a sequence set forth in SEQ ID NO:6.

10. The chimeric antigen receptor according to any one of claims 6 to 9, wherein the transmembrane domain is a transmembrane region of a protein selected from the group consisting of α, β or ζ chains of T cell receptor, CD8α, CD28, CD3ε, CD3ζ, CD45, CD4, CD5, CD9, CD16, CD22, CD33, CD37, CD64, CD80, CD86, CD134, CD137, CD154, DAP10;
preferably, the transmembrane domain comprises a transmembrane region of CD8α;
preferably, the transmembrane domain comprises a sequence set forth in SEQ ID NO:7.

11. The chimeric antigen receptor according to any one of claims 6 to 10, wherein the intracellular signaling domain comprises a primary signaling domain and optionally a costimulatory signaling domain;
preferably, the intracellular signaling domain comprises a costimulatory signaling domain and a primary signaling domain in order from the N-terminal to the C-terminal;
preferably, the intracellular signaling domain comprises a primary signaling domain and at least one costimulatory signaling domain;
preferably, the primary signaling domain comprises an immunoreceptor tyrosine activation motif (ITAM);
preferably, the primary signaling domain comprises an intracellular signaling domain of a protein selected from the group consisting of CD3ζ, FcRy, FcRβ, CD3y, CD3δ, CD3ε, CD22, CD79a, DAP10, CD79b or CD66d; preferably, the primary signaling domain comprises an intracellular signaling domain of CD3ζ; preferably, the primary signaling domain comprises a sequence set forth in SEQ ID NO: 9;
preferably, the costimulatory signaling domain comprises an intracellular signaling domain of a protein selected from the group consisting of CARD11, CD2, CD7, CD27, CD28, CD30, CD134 (OX40), CD137 (4-1BB), CD150 (SLAMF1), CD270 (HVEM), CD278 (ICOS) or DAP10; preferably, the costimulatory signaling domain is selected from an intracellular signaling domain of CD28 or an intracellular signaling domain of CD137 (4-1BB) or a combination thereof; preferably, the costimulatory signaling domain comprises a sequence set forth in SEQ ID NO: 8.

12. The chimeric antigen receptor according to any one of claims 6 to 11, wherein the chimeric antigen receptor comprises an extracellular antigen-binding domain, a spacer domain, a transmembrane domain, an intracellular signaling domain in order from the N-terminal to the C-terminal;
preferably, the spacer domain comprises a hinge region of CD8α (e.g., a sequence set forth in SEQ ID NO: 6);
preferably, the transmembrane domain comprises a transmembrane region of CD8α (e.g., a sequence set forth in SEQ ID NO: 7);
preferably, the intracellular signaling domain comprises a primary signaling domain and a costimulatory signaling domain, wherein the primary signaling domain comprises an intracellular signaling domain of CD3ζ (e.g., a sequence set forth in SEQ ID NO: 9), the costimulatory signaling domain comprises an intracellular signaling domain of CD137 (e.g., a sequence set forth in SEQ ID NO: 8);
preferably, the chimeric antigen receptor has an amino acid sequence selected from the group consisting of: (1) an amino acid sequence set forth in SEQ ID NO: 10 or 12; (2) a sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% compared to the amino acid sequence set forth in SEQ ID NO: 10 or 12.

13. An isolated nucleic acid molecule, which comprises a nucleotide sequence encoding the antibody or antigen-binding fragment thereof according to any one of claims 1 to 5, or a nucleotide sequence encoding the chimeric antigen receptor according to any one of claims 6 to 12;
preferably, the isolated nucleic acid molecule comprises a nucleotide sequence encoding the chimeric antigen receptor according to any one of claims 6 to 12, and the isolated nucleic acid molecule comprises a nucleotide sequence selected from the group consisting of: (1) a nucleotide sequence set forth in SEQ ID NO: 11 or 13; (2) a sequence having a sequence identity of at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97% %, at least 98%, at least 99%, or 100% compared to the nucleotide sequence set forth in SEQ ID NO: 11 or 13.

14. A vector, which comprises the isolated nucleic acid molecule according to claim 13;
preferably, the vector comprises a nucleotide sequence encoding the antibody or antigen-binding fragment thereof according to any one of claims 1 to 5;
preferably, the vector comprises a nucleotide sequence encoding the chimeric antigen receptor according to any one of claims 6 to 12; preferably, the vector is selected from the group consisting of DNA vector, RNA vector, plasmid, transposon vector, CRISPR/Cas9 vector, or viral vector; preferably, the vector is an expression vector; preferably, the vector is an episomal vector; preferably, the vector is a viral vector, such as a lentiviral vector, adenoviral vector or retroviral vector.

15. A host cell, which comprises the isolated nucleic acid molecule according to claim 13, or the vector according to claim 14;
preferably, the host cell comprises a nucleotide sequence encoding the antibody or antigen-binding fragment thereof according to any one of claims 1 to 5 or a vector comprising the nucleotide sequence; preferably, the host cell comprises *E. coli,* yeast, insect cell, or mammalian cell;
preferably, the host cell comprises a nucleotide sequence encoding the chimeric antigen receptor according to any one of claims 6 to 12 or a vector comprising the nucleotide sequence; preferably, the host cell comprises an immune cell (e.g., a human immune cell); more preferably, the immune cell is selected from T lymphocyte, NK cell, monocyte, macrophage or dendritic cell, and any combination thereof.

16. A method for preparing the antibody or antigen-binding fragment thereof according to any one of claims 1 to 5, which comprises, culturing the host cell according to claim 15 under conditions that allow expression of the antibody or antigen-binding fragment thereof, and recovering the antibody or antigen-binding fragment thereof from a culture of the cultured host cell; wherein the host cell comprises a nucleotide sequence encoding the antibody or antigen-binding fragment thereof according to any one of claims 1 to 5 or a vector comprising the nucleotide sequence.

17. An immune effector cell, which expresses the chimeric antigen receptor according to any one of claims 6 to 12;
preferably, the immune effector cell comprises a nucleotide sequence encoding the chimeric antigen receptor according to any one of claims 6 to 12 or a vector comprising the nucleotide sequence;
preferably, the immune effector cell is selected from the group consisting of T lymphocyte, NK cell, monocyte, macrophage or dendritic cell and any combination thereof;
preferably, the immune effector cell is selected from T lymphocyte and/or NK cell.

18. The method for preparing the immune effector cell according to claim 17, which comprises: (1) providing an immune effector cell; (2) introducing the isolated nucleic acid molecule according to claim 13 or the vector according to claim 14 into the immune effector cell; wherein the isolated nucleic acid molecule or vector comprises a nucleotide sequence encoding the chimeric antigen receptor according to any one of claims 6 to 12;
preferably, the immune effector cell is selected from the group consisting of T lymphocyte, NK cell, monocyte, dendritic cell, macrophage and any combination thereof; preferably, the immune effector cell is selected from T lymphocyte and/or NK cell;
preferably, in step (1), the immune effector cell is subjected to a pretreatment, and the pretreatment comprises sorting, activation and/or proliferation of the immune effector cell; preferably, the pretreatment comprises contacting the immune effector cell with an anti-CD3 antibody and an anti-CD28 antibody;
preferably, in step (2), the nucleic acid molecule or vector is introduced into the immune effector cell by viral infection;
preferably, in step (2), the nucleic acid molecule or vector is introduced into the immune effector cell by means of non-viral vector transfection, such as calcium phosphate transfection, DEAE-dextran-mediated transfection, microinjection, transposon vector system, CRISPR/Cas9 vector, TALEN method, ZFN method or electroporation method;
preferably, a step of expanding the immune effector cell obtained in step (2) is further comprised after step (2).

19. A test kit, which comprises the isolated nucleic acid molecule according to claim 13 or the vector according to claim 14;
preferably, the isolated nucleic acid molecule or vector comprises a nucleotide sequence encoding the chimeric antigen receptor according to any one of claims 6 to 12.

20. Use of the kit according to claim 19 in the manufacture of a chimeric antigen receptor that specifically binds to BCMA or a cell expressing the chimeric antigen receptor;
preferably, the cell is an immune effector cell, such as T lymphocyte and/or NK cell.

21. A conjugate, which comprises the antibody or antigen-binding fragment thereof according to any one of claims 1 to 5 and a modification moiety linked to the antibody or antigen-binding fragment thereof;
preferably, the modification moiety is selected from a detectable label, such as enzyme, radionuclide, fluorescent dye, luminescent substance (e.g., chemiluminescent substance) or biotin;
preferably, the modification moiety is selected from a therapeutic agent, such as an anti-tumor activity drug or a cytotoxic agent.

22. A pharmaceutical composition, which comprises the antibody or antigen-binding fragment thereof according to any one of claims 1 to 5, or the chimeric antigen receptor according to any one of claims 6 to 12, or the isolated nucleic acid molecule according to claim 13, or the vector according to claim 14, or the host cell according to claim 15, or the immune effector cell according to claim 17, or the conjugate according to claim 21, and a pharmaceutically acceptable carrier and/or excipient;
preferably, the pharmaceutical composition further comprises an additional pharmaceutically active agent; preferably, the additional pharmaceutically active agent is a drug with anti-tumor activity, such as alkylating agent, mitotic inhibitor, anti-tumor antibiotic, anti-metabolite, topoisomerase inhibitor, tyrosine kinase inhibitor, radionuclide agent, radiosensitizer, anti-angiogenic agent, cytokine, antibody that specifically targets a tumor cell, or immune checkpoint inhibitor.

23. Use of the antibody or antigen-binding fragment thereof according to any one of claims 1 to 5, or the chimeric antigen receptor according to any one of claims 6 to 12, or the isolated nucleic acid molecule according to claim 13, or the vector according to claim 14, or the host cell according to claim 15, or the immune effector cell according to claim 17, or the conjugate according to claim 21, or the pharmaceutical combination according to claim 22, in the manufacture of a medicament for the prevention and/or treatment of a B cell-related disease in a subject (e.g., a human);
preferably, the B-related disease is a B-cell malignancy, for example, multiple myeloma (MM) or non-Hodgkin's lymphoma (NHL);
preferably, the B cell-related disease is an autoimmune disease, such as systemic lupus erythematosus, rheumatoid arthritis, idiopathic thrombocytopenic purpura or myasthenia gravis or autoimmune hemolytic anemia;
preferably, the B cell-related disease is selected from the group consisting of multiple myeloma, non-Hodgkin's lymphoma, B cell proliferation of uncertain malignant potential, lymphomatoid granulomatosis, post-transplant lymphoproliferative disease, immunomodulatory disorder, rheumatoid arthritis, myasthenia gravis, idiopathic thrombocytopenic purpura, antiphospholipid syndrome, Chagas' disease, Graves' disease, Wegener's granulomatosis, polyarteritis nodosa, Sjogren's syndrome, pemphigus vulgaris, scleroderma, multiple sclerosis, antiphospholipid syndrome, ANCA-associated small vessel vasculitis, Goodpasture's disease, Kawasaki disease, autoimmune hemolysis anemia and rapidly progressive glomerulonephritis, heavy chain disease, primary or immune cell-associated amyloidosis, or monoclonal gammopathy of undetermined significance.

24. A method for preventing and/or treating a B cell-related disease in a subject (e.g., a human), the method comprising administering to the subject in need thereof an effective amount of the antibody or antigen-binding fragment thereof according to any one of claims 1 to 5, or the immune effector cell according to claim 17, or the conjugate according to claim 21, or the pharmaceutical combination according to claim 22,
preferably, the B-related condition is a B-cell malignancy, for example, multiple myeloma (MM) or non-Hodgkin's lymphoma (NHL);
preferably, the B cell-related disease is an autoimmune disease, such as systemic lupus erythematosus, rheumatoid arthritis, idiopathic thrombocytopenic purpura or myasthenia gravis or autoimmune hemolytic anemia;
preferably, the B cell-related disease is selected from the group consisting of multiple myeloma, non-Hodgkin's lymphoma, B cell proliferation of uncertain malignant potential, lymphomatoid granulomatosis, post-transplant lymphoproliferative disease, immunomodulatory disorder, rheumatoid arthritis, myasthenia gravis, idiopathic thrombocytopenic purpura, antiphospholipid syndrome, Chagas' disease, Graves' disease, Wegener's granulomatosis, polyarteritis nodosa, Sjogren's syndrome, pemphigus vulgaris, scleroderma, multiple sclerosis, antiphospholipid syndrome, ANCA-associated small vessel vasculitis, Goodpasture's disease, Kawasaki disease, autoimmune hemolysis anemia and rapidly progressive glomerulonephritis, heavy chain disease, primary or immune cell-associated amyloidosis, or monoclonal gammopathy of undetermined significance.

25. A method for preventing and/or treating a B cell-related disease in a subject (e.g., a human), the method comprising the steps of:
(1) providing an immune effector cell;
(2) introducing an isolated nucleic acid molecule or vector comprising a nucleotide sequence encoding the chimeric antigen receptor according to any one of claims 6 to 12 into the immune effector cell described in step (1) to obtain an immune effector cell expressing the chimeric antigen receptor;
(3) administering the immune effector cell obtained in step (2) to the subject for treatment;
preferably, the immune effector cell is selected from the group consisting of T lymphocyte, NK cell, monocyte, macrophage, dendritic cell, or any combination of these cells;
preferably, prior to step (1), the method further comprises a step of obtaining the immune effector cell from the subject.

26. A method for diagnosing whether a subject (e.g., a human) suffers from a BCMA-expressing tumor, which comprises detecting an amount of BCMA in a sample from the subject by using the antibody or antigen-binding fragment thereof according to any one of claims 1 to 5;
preferably, the method further comprises a step of comparing the amount of BCMA in the sample from the subject to a reference value;
preferably, the amount of BCMA in the sample from the subject is detected by:
(1) contacting the sample from the subject with the antibody or antigen-binding fragment thereof according to any one of claims 1 to 5;
(2) detecting an amount of a complex formed by the antibody or antigen-binding fragment thereof and BCMA;
preferably, in step (1), the antibody or antigen-binding fragment thereof further bears a detectable label;
preferably, the sample is selected from the group consisting of urine, blood, serum, plasma, saliva, ascites, circulating cell, circulating tumor cell, non-tissue-associated cell, tissue (e.g., surgically resected tumor tissue, biopsy or fine needle aspiration tissue), histological preparation;
preferably, the BCMA-expressing tumor is selected from B-cell malignancies, such as multiple myeloma (MM) or non-Hodgkin's lymphoma (NHL);
preferably, the method further comprises administering a BCMA-targeted immunotherapy to the subject diagnosed with a BCMA-expressing tumor; preferably, the BCMA-targeted immunotherapy is the method according to claim 24 or 25.
